(19) Europäisches Patentamt
European Patent Office
Office européen des brevets

(11) **EP 4 035 552 B1**

(12) **EUROPEAN PATENT SPECIFICATION**

(45) Date of publication and mention
of the grant of the patent:
**01.11.2023  Bulletin 2023/44**

(21) Application number: **22153975.2**

(22) Date of filing: **28.01.2022**

(51) International Patent Classification (IPC):
*A43B 7/00* (2006.01)        *A43B 7/12* (2006.01)
*A61K 9/00* (2006.01)        *A61P 17/00* (2006.01)
*A61P 31/04* (2006.01)

(52) Cooperative Patent Classification (CPC):
**A43B 7/00; A43B 7/125; A61K 9/0014;**
**A61P 17/00; A61P 31/04**

(54) **HYPOALLERGENIC FOOTWEAR AND METHOD FOR PREVENTING ALLERGIC CONTACT DERMATITIS ACD**

HYPOALLERGENES SCHUHWERK UND VERFAHREN ZUR VERHINDERUNG VON ALLERGISCHER KONTAKTDERMATITIS

CHAUSSURE HYPOALLERGÉNIQUE ET PROCÉDÉ DE PRÉVENTION DE LA DERMATITE ALLERGIQUE DE CONTACT (ACD)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.02.2021  IT 202100002126**

(43) Date of publication of application:
**03.08.2022  Bulletin 2022/31**

(73) Proprietor: **Binar S.r.l.**
**20123 Milano (MI) (IT)**

(72) Inventor: **BIANCHI, Enio Angelo**
**27029 VIGEVANO (PV) (IT)**

(74) Representative: **PGA S.p.A.**
**Via Mascheroni, 31**
**20145 Milano (IT)**

(56) References cited:
WO-A1-2012/065792       US-A- 4 836 217
US-A1- 2011 162 239      US-A1- 2019 262 456

**Description**

FIELD OF THE INVENTION

The present invention relates to a method for preventing allergic contact dermatitis ACD in a user's foot.

The present invention also relates to a hypoallergenic footwear including a combination of structural and chemical features which allow to prevent onset of allergic contact dermatitis ACD.

Furthermore, the present invention also relates to the use by a user of hypoallergenic footwear, i.e. hypoallergenic shoes, to prevent allergic contact dermatitis ACD.

BACKGROUND

The ACD is an allergic reaction of the human skin due to contact with sensitizing chemical substances, called allergens, able to cause skin inflammation, such as redness and persistent itching. The ACD is a pathology detected through a diagnostic tool, i.e. a patch test, which patients are subjected to. The patch test involves the contact of the sensitized part of the patient with a plurality of materials, each of them containing a specific allergen. The positive reaction to one or more of these substances indicates the cause of the sensitization.

It is known that there is a part of the allergic population that, although showing symptoms, has never undergone an allergy test.

The onset of ACD finds a favorable condition inside the footwear, because of presence of moisture in an occlusive environment, making the foot particularly vulnerable to the development of dermatitis. Inflammation occurs in the form of abrasions that appear mainly near the upper part of the toes. Such inflammation may easily complicate with bacterial infections, especially in diabetic users.

Normally, the ACD may be treated by avoiding contacts with the responsible substance.

US 2011/162239 A1 relates to a method for preventing allergic reactions comprising vapour permeable structures inhibiting growth by means of the hypo-allergenic structure.

SCOPE OF THE INVENTION

The scope of this invention is therefore to provide a method, according to the present invention, able to prevent or reduce the risk of ACD onset in a user's foot.

A further scope is to provide use of hypoallergenic footwear according to the present invention to prevent onset of ACD in a user's foot.

A further scope is to detect a feature combination, in terms of hypoallergenic materials to be used, traspirability and dermo-compatibility, able to prevent or reduce the risk of onset of ACD in a user's foot.

A further scope is to provide a footwear which is both comfortable and resistant.

These scopes and more, which will appear more from the following description, are substantially achieved by a method, use, and a footwear in accordance with one or more of the following claims and/or aspects.

SUMMARY SECTION

A first aspect refers to a method for preventing allergic contact dermatitis ACD in a user's foot by wearing a hypoallergenic footwear (10), wherein said footwear comprises at least an upper (1), an insole (2) and an outsole (3).

A 2nd aspect refers to a hypoallergenic footwear (10) for preventing allergic contact dermatitis ACD, wherein said footwear comprises at least an upper (1), an insole (2) and an outsole (3).

In a 3rd aspect according to the preceding aspects, at least the upper (1) and the insole (2) have hypoallergenic structure according to a predefined list of allergenic materials and corresponding maximum concentrations, said list comprising:

- chromium salt, the chromium salt being present at most in a concentration lower than 0,01 mg/kg according to DIN 54233-3:2010;
- dimethyl fumarate, the dimethyl fumarate being present at most in a concentration lower than 0,01 mg/kg according to EN ISO 16186:2012;
- para tertiary butylphenol formaldehyde, the para tertiary butylphenol formaldehyde being present at most in a concentration lower than 5 mg/kg according to EN ISO 14184-1:2011;
- formaldehyde, the formaldehyde being present at most in a concentration lower than 5 mg/kg according to EN ISO 14184-1:2011;
- chromium, soluble total, the chromium being present at most in a concentration lower than 0,01 mg/kg according to DIN 54233-3:2010.

In a 4th aspect according to any one of the preceding aspects, said footwear (10), in particular at least the upper (1), defines a permeable structure having:

- a water vapour permeability equal to or higher than 2,0 mg/(cm²·h), in particular higher than 13 mg/(cm²·h), according to EN 13515:2001/ISO 17 699:2003; and/or
- a water vapour coefficient equal to or higher than 20 mg/cm², in particular higher than 100 mg/cm², according to EN 13515:2001/ISO 17 699:2003.

In a 5th aspect according to any one of the preceding aspects, at least the upper (1) and the insole (2) have a dermocompatibility defined by a growth inhibition percentage equal to or lower than 30%, in particular lower than 25%, according to DIN EN ISO 10993, DIN EN ISO 10993-5,

or wherein the footwear (10) includes a lining (4), and wherein at least said lining (4) and the insole (2) have a dermocompatibility defined by a growth inhibition percentage equal to or lower than 30%, in particular lower than 25%, according to DIN EN ISO 10993, DIN EN ISO 10993-5,

or wherein the footwear (10) includes a lining (4), and wherein at least said lining (4), upper (1) and the insole (2) have a dermocompatibility defined by a growth inhibition percentage equal to or lower than 30%, in particular lower than 25%, according to DIN EN ISO 10993, DIN EN ISO 10993-5.

In a 6th aspect according to any one of the preceding aspects, the footwear (10) includes a lining (4) comprising the hypoallergenic structure according to said list of predefined maximum concentrations of allergenic materials, said lining (4) contacting the user's foot when the hypoallergenic footwear (10) is in use.
In an aspect 6bis according to any one of the preceding aspects, the footwear (10) includes a lining (4) having a dermocompatibility defined by a growth inhibition percentage equal to or lower than 30%, in particular lower than 25%, according to DIN EN ISO 10993, DIN EN ISO 10993-5.
In a 7th aspect according to any one of the preceding aspects, said predefined list of allergenic materials and corresponding maximum concentrations further includes at least one between, optionally all:

- potassium dichromate, the potassium dichromate being present at most in a concentration lower than 0,01 mg/kg as Cr VI according to acid digestion and Inductively Coupled Plasma Emission Spectroscopy (ICP-OES) methods;
- cobalt chloride, the cobalt chloride being present at most in a concentration lower than 0,01 mg/kg as Co according to acid digestion and Inductively Coupled Plasma Emission Spectroscopy (ICP-OES) methods;
- nickel sulfate, the nickel sulfate being present at most in a concentration lower than 0,01 mg/kg as Ni according to acid digestion and Inductively Coupled Plasma Emission Spectroscopy (ICP-OES) methods.

In a 8th aspect according to any one of the preceding aspects, the upper (1) and the insole (2) contact with the user's foot when the hypoallergenic footwear (10) is in use.
In a 9th aspect according to any one of the preceding aspects, said list of allergenic materials of the upper (1) and the insole (2), optionally of the lining (4), insole (2) and preferably also of the upper (1), further includes at least one between, optionally all the following substances:

- paraphenylenediamine, the paraphenylenediamine being present at most in a concentration lower than 0,5 mg/kg according to solvent extraction and/or Gas Chromatography Mass Spectrometry (GC-MS) and/or Liquid Chromatography with UltraViolet LC-UV determination;
- an organotin compound, the organotin compound being present at most in a concentration lower than 0,02 mg/kg as Ni according to CEN ISO/TS 16179:2012;
- azo dyes, the azo dyes being present at most in a concentration lower than 5 mg/kg according to UNI EN 14362

In a 10th aspect according to any one of the preceding aspects, said organotin compound includes one or more of, optionally all, the following substances:

- Tributyltin (TBT) in a concentration lower than 0,02 mg/kg;
- Monobutyltin (MBT) in a concentration lower than 0,02 mg/kg;
- Monooctylzinn (MOT) in a concentration lower than 0,02 mg/kg;
- Dibutyltin (DBT) in a concentration lower than 0,02 mg/kg;

- Dibutyltin dichloride (DBTC) in a concentration lower than 0,02 mg/kg;
- Dioctyltin (DOT) in a concentration lower than 0,02 mg/kg;
- Triphenyltin (TPhT) in a concentration lower than 0,02 mg/kg;
- Bis (Tributyltin) oxide (TBTO) in a concentration lower than 0,02 mg/kg;
- Dipheyltin (DPhT) in a concentration lower than 0,02 mg/kg;
- Dipropyltin (DPT) in a concentration lower than 0,02 mg/kg;
- Monopheyltin (MPhT) in a concentration lower than 0,02 mg/kg;
- Tetrabutyltin (TeBT) in a concentration lower than 0,02 mg/kg;
- Tricyclohexyltin (TCyHT) in a concentration lower than 0,02 mg/kg;
- Trimethyltin (TMT) in a concentration lower than 0,02 mg/kg;
- Trioctyltin (TOT) in a concentration lower than 0,02 mg/kg;
- Tripropyltin (TPT) in a concentration lower than 0,02 mg/kg.

In a 11th aspect according to any one of the preceding aspects, the footwear (10) further comprises a reinforcement (5) including a concentration of said organotin compound lower than 0,02 mg/kg as Ni according to CEN ISO/TS 16179:2012.

In a 12th aspect according to any one of the preceding aspects, the outsole (3) has:

- a structure comprising the same predefined list of allergenic materials and corresponding maximum concentrations of the upper (1) and the insole (2), in particular of the upper (1), insole (2) and of the lining (4), in particular said list of allergenic materials comprising at least one between:

  ○ chromium salts, the chromium salts being present at most in a concentration lower than 0,01 mg/kg according to DIN 54233-3:2010;
  ○ dimethyl fumarate, said dimethyl fumarate being present at most in a concentration lower than 0,01 mg/kg according to EN ISO 16186:2012;
  ○ Para tertiary butylphenol formaldehyde, said Para tertiary butylphenol formaldehyde being present at most in a concentration lower than 5 mg/kg according to EN ISO 14184-1:2011;
  ○ formaldehyde, said formaldehyde being present at most in a concentration lower than 5 mg/kg according to EN ISO 14184-1:2011;
  ○ a chromium, soluble total, concentration lower than 0,01 mg/kg according to DIN 54233-3:2010;
  ○ potassium dichromate, said potassium dichromate being present at most in a concentration lower than 0,01 mg/kg as Cr VI according to acid digestion and Inductively Coupled Plasma Emission Spectroscopy (ICP-OES) methods;
  ○ cobalt chloride, said cobalt chloride being present at most in a concentration lower than 0,01 mg/kg as Co according to acid digestion and Inductively Coupled Plasma Emission Spectroscopy (ICP-OES) methods;
  ○ nickel sulfate, said nickel sulfate being present at most in a concentration lower than 0,01 mg/kg as Ni according to acid digestion and Inductively Coupled Plasma Emission Spectroscopy (ICP-OES) methods.
  ○ paraphenylenediamine, said paraphenylenediamine being present at most in a concentration lower than 0,5 mg/kg according to solvent extraction and/or Gas Chromatography Mass Spectrometry (GC-MS) and/or Liquid Chromatography with UltraViolet LC-UV determination;
  ○ organotin compounds, said organotin compounds being present at most in a concentration lower than 0,02 mg/kg as Ni according to CEN ISO/TS 16179:2012;
  ○ azo dyes, said azo dyes being present at most in a concentration lower than 5 mg/kg according to UNI EN 14362.

  or

- a second distinct list of allergenic materials including all or at least one substance between:

  ○ chromium salts, said chromium salts being present at most in a concentration lower than 0,01 mg/kg according to DIN 54233-3:2010;
  ○ dimethyl fumarate, said dimethyl fumarate being present at most in a concentration lower than 0,01 mg/kg according to EN ISO 16186:2012;
  ○ Para tertiary butylphenol formaldehyde, said Para tertiary butylphenol formaldehyde being present at most in a concentration lower than 5 mg/kg according to EN ISO 14184-1:2011;
  ○ formaldehyde, said formaldehyde being present at most in a concentration lower than 5 mg/kg according to EN ISO 14184-1:2011;
  ○ chromium, soluble total, said chromium soluble total being present at most in a concentration lower than 0,01 mg/kg according to DIN 54233-3:2010.

In a 13th aspect according to the preceding aspect, said second distinct list of allergenic materials further includes all or at least one substance between:

- a potassium dichromate concentration lower than 0,01 mg/kg as Cr VI according to acid digestion and Inductively Coupled Plasma Emission Spectroscopy (ICP-OES) methods;
- a cobalt chloride concentration lower than 0,01 mg/kg as Co according to acid digestion and Inductively Coupled Plasma Emission Spectroscopy (ICP-OES) methods;
- a nickel sulfate concentration lower than 0,01 mg/kg as Ni according to acid digestion and Inductively Coupled Plasma Emission Spectroscopy (ICP-OES) methods;
- a paraphenylenediamine concentration lower than 0,5 mg/kg according to solvent extraction and/or Gas Chromatography Mass Spectrometry (GC-MS) and/or Liquid Chromatography with UltraViolet LC-UV determination;
- an organotin compounds concentration lower than 0,02 mg/kg as Ni according to CEN ISO/TS 16179:2012;
- a azo dyes concentration lower than 5 mg/kg according to UNI EN 14362;

In a 14th aspect according to any one of the preceding aspects, the outsole (3) has a dermocompatibility higher than 30%.

In a 15th aspect according to any one of the preceding aspects, the predefined list of allergenic materials and corresponding maximum concentrations of the upper (1), in particular of the upper (1), lining (4) and insole (2), of the footwear (10) further includes both or at least one between:

- aromatic amines, said aromatic amines being present at most in a concentration lower than 5 mg/kg according to EN 14362-1:2017 or EN14362-2:2003/AC:2005 or EN 14362-3:2017; and
- heavy metal(s), said heavy metal(s) being present at most in concentration, in particular soluble heavy metal concentration, lower than 0,1 mg/kg according to DIN 54233-3:2010 and/or EN1122:2001 and/or acid digestion and/or Inductively Coupled Plasma Emission Spectroscopy (ICP-OES) method.

In a 16th aspect according to the preceding aspect, the footwear (10) further includes a collar (6) and/or a reinforcement (5) comprising said aromatic amines in a concentration lower than 5 mg/kg according to EN 14362-1:2017 or EN14362-2:2003/AC:2005 or EN 14362-3:2017, and/or said heavy metal(s) in a concentration lower than 0,1 mg/kg according to DIN 54233-3:2010 and/or EN1122:2001 and/or acid digestion and/or Inductively Coupled Plasma Emission Spectroscopy (ICP-OES) method.

In a 17th aspect according to any one of the two preceding aspects, the aromatic amines comprise all or at least one substance between:

- Benzidine, said benzidine being present at most with a concentration lower than 5 mg/kg;
- o-Anisidine, said o-Anisidine being present at most with a concentration lower than 5 mg/kg;
- o-aminoazotoluene, said o-aminoazotoluene being present at most with a concentration lower than 5 mg/kg;
- o-toluidine, said o-toluidine being present at most with a concentration lower than 5 mg/kg;
- p-Cresidine, said p-Cresidine being present at most with a concentration lower than 5 mg/kg;
- p-Chloroaniline, said Chloroaniline being present at most with a concentration lower than 5 mg/kg;
- 2-Amino-4-Nitrotoluene, said 2-Amino-4-Nitrotoluene being present at most with a concentration lower than 5 mg/kg;
- 2-Naphthylamine, said 2-Naphthylamine being present at most with a concentration lower than 5 mg/kg;
- 2,4-Toluylenediamine, said 2,4-Toluylenediamine being present at most with a concentration lower than 5 mg/kg;
- 2,4 Xylidine, said 2,4 Xylidine being present at most with a concentration lower than 5 mg/kg;
- 2,6 Xylidine, said 2,6 Xylidine being present at most with a concentration lower than 5 mg/kg;
- 3,3' - Dimethylbenzidine, said 3,3' Dimethylbenzidine being present at most with a concentration lower than 5 mg/kg;
- 3,3' - Dichlorobenzidine, said 3,3' Dichlorobenzidine being present at most with a concentration lower than 5 mg/kg;
- 3,3' - Dimethoxybenzidine, said 3,3' Dimethoxybenzidine being present at most with a concentration lower than 5 mg/kg;
- 3,3' - Dimethyl - 4,4' - Diaminodiphenylmethane, said 3' - Dimethyl - 4,4' - Diaminodiphenylmethane being present at most with a concentration lower than 5 mg/kg
- 4 - Aminodiphenyl, said 4 - Aminodiphenyl being present at most with a concentration lower than 5 mg/kg;
- 4 - Chloro-O-Toluine, said 4 - Chloro-O-Toluine being present at most with a concentration lower than 5 mg/kg;
- 4,4' - Diaminodiphenylmethane, said 4,4' - Diaminodiphenylmethane being present at most with a concentration lower than 5 mg/kg;
- 4,4' - Methylene-Bis-(2-Chloroaniline), said 4,4' - Methylene-Bis-(2-Chloroaniline) being present at most with a concentration lower than 5 mg/kg;
- 4,4' - Thiodianiline, said Thiodianiline being present at most with a concentration lower than 5 mg/kg;

- 4,4' - Oxydianiline, said 4,4' - Oxydianiline being present at most with a concentration lower than 5 mg/kg;
- 2,4,5 - Trimethylaniline, said 2,4,5 - Trimethylaniline being present at most with a concentration lower than 5 mg/kg;
- 4 - Aminoazobenzene, said 4 - Aminoazobenzene being present at most with a concentration lower than 5 mg/kg.

In a 18th aspect according to any one of the three preceding aspects, said heavy metals include all or at least one between a:

- Arsenic (As) concentration lower than 0,01 mg/kg;
- Cadmium (Cd) concentration lower than 0,01 mg/kg;
- Chromium concentration lower than 0,01 mg/kg;
- Cobalt (Co) concentration lower than 0,01 mg/kg;
- Lead (Pb) concentration lower than 0,01 mg/kg;
- Mercury (Hg) concentration lower than 0,01 mg/kg;
- Nickel (Ni) concentration lower than 0,01 mg/kg.

In a 19th aspect according to any one of the preceding aspects, at least one between the upper (1), the lining (4) and the insole (2) of the footwear (10) has a pH value comprised between 5 and 7,5 according to EN ISO 3071 :2020, in particular a pH value comprised between 5,5 and 7.

In a 20th aspect according to any one of the preceding aspects, the footwear (10) further comprises a collar (6) and a reinforcement (5) both having a pH value comprised between 5 and 7,5 according to EN ISO 3071 :2020, in particular a pH value comprised between 5,5 and 7.

In a 21st aspect according to any one of the preceding aspects, the list of allergenic materials and corresponding concentrations and/or the second distinct list of allergenic materials include all or at least one substance between:

- mercapto mix, said mercapto mix being present at most with a concentration lower than 0,1 mg/kg according to solvent extraction and determination in *HPLC-UV*;
- carba mix, said carba mix being present at most with a concentration lower than 0,1 mg/kg according to ...;
- Thiuram Mix, said Thiuram Mix being present at most with a concentration lower than 0,1 mg/kg;
- Mercaptobenziotaziolo, said Mercaptobenziotaziolo being present at most with a concentration lower than 0,1 mg/kg;
- Mixed Dialkil Thioureas, said Mixed Dialkil Thioureas being present at most with a concentration lower than 0,1 mg/kg;
- Black Rubber Mix, said Black Rubber Mix being present at most with a concentration lower than 0,1 mg/kg;
- Parabeni Mix, said Parabeni Mix being present at most with a concentration lower than 0,1 mg/kg.

In a 22nd aspect according to the preceding aspect, the mercapto mix comprises all or at least one substance in the group including:

- N-Cyclohexylbenzothiazyl-sulfenamide
- cyclohexyl benzothiazolesulfenamide
- 2(cyclohexylaminothio)benzothiazole
- benzothiazyl-2-cyclohexylsulfenamide
- Dibenzothiazyl disulfide
- 2,2'-cithiobis(benzothiazole)
- dibenzothiazolyl disulfide
- 2,2'bis(benzothiazolyl) disulfide
- 2,2'-dibenzothiazyl disulfide
- 2-mercaptobenzothiazole disulfide
- Morpholinylmercaptobenzothiazole
- 2-benzothiazolyl morpholino disulfide
- 2morpholinodithiobenzothiazole
- benzothiazole 2-(4-morpholinyl)
- 4-morpholinyl 2-benzothiazyl disulfide
- Benzothiazole,2-(4-morpholinyldithio).

In a 23rd aspect according to any one of the two preceding aspects, the carba mix comprises all or at least one substance in the group including:

- Diphenylguanidine
- 1,3-Diphenylguanidine

- N,N'-Diphenylguanidine
- Zincdibutyldithiocarbamate
- bis (N,N-dibutyldithiocarbamato) zinc
- carbamic acid dibutyldithio-, zinc complex
- zinc bis (dibutyldithiocarbamate)
- Zincdiethyldithiocarbamate
- diethyldithiocarbamic acid zinc salt
- zinc bis (diethyldithiocarbamate)
- zinc diethylcarbamodithioate.

In a 24th aspect according to any one of the preceding aspects, the footwear (10) further comprises a reinforcement (5) according to said predefined list of allergenic materials and corresponding maximum concentrations.

In a 25th aspect according to any one of the preceding aspects, the footwear (10) further comprises a collar (6) according to said predefined list of allergenic materials and corresponding maximum concentrations.

In a 26th aspect according to any one of the preceding aspects, the soluble heavy metals further comprises elements having atomic number higher than 20, in particular higher than 23, or a density higher than 5 $g/cm^3$.

In a 27th aspect according to any one of the preceding aspects, the permeable structure has a water vapour absorption equal to or higher than 1 $mg/cm^2$, in particular higher than 1,5 $mg/cm^2$, according to EN 13515:2001/ISO 17 699:2003.

In a 28th aspect according to any one of the preceding aspects, the upper has an ultimate strength higher than or equal to 500 N/50mm according to UNI EN ISO 13934-1:2013, in particular higher than 1000 N/50mm.

In a 29th aspect according to any one of the preceding aspects, the upper has an ultimate elongation at failure higher than or equal to 15% according to UNI EN ISO 13934-1:2013, in particular higher than 40%, more in particular higher than 55%.

In a 30th aspect according to any one of the preceding aspects, the dermocompatibility defined by growth inhibition percentage is computed by the following formula:

$$\%WH = 100 - 100 \cdot \frac{(OD_{570nm} sample) - (OD_{570nm} blank)}{(OD_{570nm} control) - (OD_{570nm} blank)}$$

wherein:

$OD_{570nm}$ = optical density quantified at 570 nm wavelengths;
$OD_{570nm}$ Sample = Average extinction of a sample dilution out of 3 parallel wells;
$OD_{570nm}$ Blank = Average extinction of a blank dilution out of 3 parallel wells, the blank dilution being without cells;
$OD_{570nm}$ Control = Average extinction of the solvent control out of 3 parallel wells.

In a 31st aspect according to any one of the preceding aspects, the upper (1), in particular the upper (1) and the lining (4), are based on, and particularly made of, nylon or polyester or a combination thereof.

In a 32nd aspect according to any one of the preceding aspects, the upper (1), in particular the upper (1) and the lining (4), does/do not comprise animal-derived materials.

In a 33th aspect according to any one of the preceding aspects, the water vapour permeability and/or the water vapour coefficient of the permeable structure are referred to the footwear (10) in a use condition, in particular to the footwear (10) including the upper (1), the insole (2), the outsole (3), the collar (6), the reinforcement (5) and optionally the lining (4),
or wherein the water vapour permeability and/or the water vapour coefficient of the permeable structure are referred only to the upper and the lining.

A 34th aspect relates to a hypoallergenic footwear (10):

- according to the predefined list of allergenic materials according to anyone of the preceding aspects;
- comprising the permeable structure according to anyone of the preceding aspects;
- and having a dermocompatibility according to anyone of the preceding aspects.

A 35th aspect relates to the use of a hypoallergenic footwear (10) for preventing allergic contact dermatitis ACD in a user's foot, said footwear (10) being according to any one of the preceding aspects.

<u>DRAWINGS</u>

Some embodiments and some aspects of the invention will be described below with reference to the attached drawings, provided for illustrative purposes only, wherein:

- Figures 1 is a perspective views of a footwear according to an embodiment of the present invention;
- Figure 2 is a cross-sectional view of the footwear of figure 1.

<u>DEFINITIONS</u>

In this detailed description, corresponding parts illustrated in the various figures are indicated with the same numerical references. The figures may illustrate the invention by means of non-scale representations; therefore, parts and components illustrated in the figures relating to the object of the invention may relate exclusively to schematic representations.

<u>DETAILED DESCRIPTION</u>

**Hypoallergenic footwear**

A detailed description of a hypoallergenic footwear able to prevent onset of allergic contact dermatitis ACD in a user's foot is provided. The present invention relates to the main features that a footwear should fulfill in order to be able to prevent, or reduce the risk of, the onset of ACD in a user's foot.

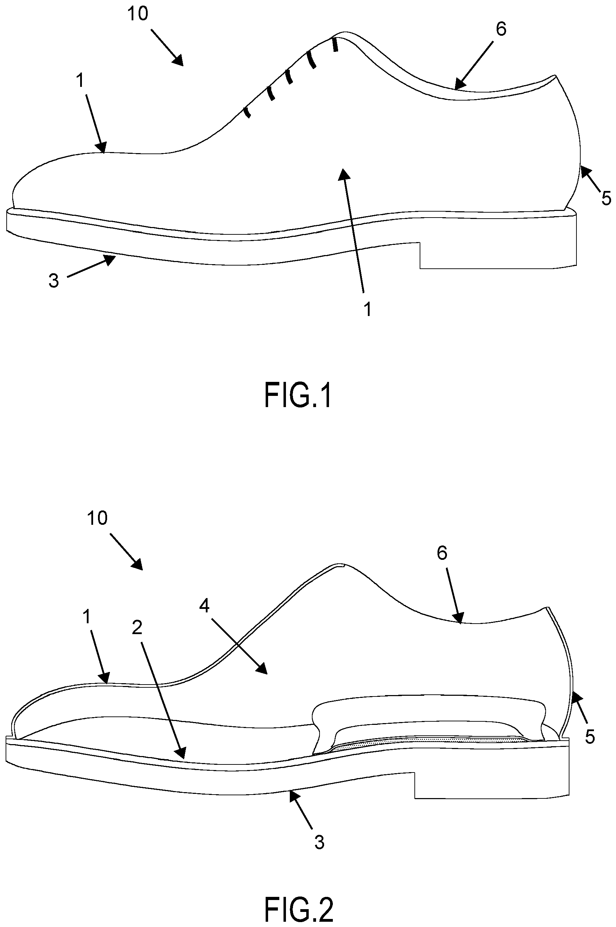

The footwear 10 may be i.e. a shoe, a sandal or a slipper comprising at least an upper 1, an insole 2 and an outsole 3. A schematic representation of a shoe according to the present invention is depicted in figures 1 and 2: the shoe may be any kind of shoe, namely a classic shoe or a sneaker.

The upper 1 is defined as the entire part of the footwear 10 that covers the foot above the outsole 3. The upper 1 of the shoe may also comprise the vamp 1a defining the front portion of the shoe, the quarters 1b defining the sides and back of the shoe, and an inner lining 4.

The upper 1 may also comprise a reinforcement 5, placed in a back and/or front portions of the footwear, configured to surround the user's heel and toe puff in a use condition. The reinforcement has a structural stiffness higher than a correspondent stiffness of the structure of the upper: the reinforcement may be made of plastic polymers or a textile material.

The footwear, or the upper itself, may optionally include the lining 4 arranged inside the shoe. The lining 4 aims to improve comfort, breathability, and to increase the lifespan of the shoe and thermal insulation as well.

The insole 2 is defined as a layer sitting inside the footwear 10 and configured to support the user's foot during a use condition: the insole 2 should provide a comfortable support to the user's foot. The insole may have a thickness comprised between 1 mm and 15 mm: the thickness may also vary along the longitudinal extension of the insole from the front portion to a back portion, wherein the latter is configured to support the user's heel. For example, this back portion of the insole may have a higher thickness than the front portion.

The outsole 3 is defined as the portion of the footwear configured to contact with the ground. The outsole 3 should provide grip, durability, and, optionally, water resistance and/or perspiration.

According to the present invention, at least the upper 1 and the insole 2 have hypoallergenic structure according to a predefined list of allergenic materials and corresponding maximum concentrations, wherein this list comprises:

- chromium salt, the chromium salt being present at most in a concentration lower than 0,01 mg/kg according to DIN 54233-3:2010;
- dimethyl fumarate, the dimethyl fumarate being present at most in a concentration lower than 0,01 mg/kg according to EN ISO 16186:2012;
- para tertiary butylphenol formaldehyde, the para tertiary butylphenol formaldehyde being present at most in a concentration lower than 5 mg/kg according to EN ISO 14184-1:2011;
- formaldehyde, the formaldehyde being present at most in a concentration lower than 5 mg/kg according to EN ISO 14184-1:2011;
- chromium, soluble total, the chromium being present at most in a concentration lower than 0,01 mg/kg according to DIN 54233-3:2010.

The chromium and chromium salts, dimethyl fumarate, para tertiary butylphenol formaldehyde, formaldehyde are considered as high ranking allergenic materials which may substantially contribute to the onset of the ACD when placed in contact to a user's foot. Notably, the term "*contact*" is used in a broad manner, namely considering both direct and indirect contact. In case of direct contact, the foot skin directly contacts the shoe structure, while in case of indirect contact the foot skin may be separated by the shoe structure by a thin layer, such as a sock.

The list presented above and in the appended claims defines the requirements in terms of maximum concentrations of the mentioned allergenic materials: therefore, this list does not imply the presence, although very low, of these materials in the shoe, while it specifies the maximum concentration acceptable for these allergenic materials. A concentration higher than the limits specified in the list would compromise the hypoallergenic property of the claimed footwear, leading to higher probabilities of ACD onset: on the other hand, a concentration comprised between 0 (zero) mg/kg and the limit specified in the list for each of the mentioned allergenic material is considered to be acceptable.

In case the footwear comprises a lining 4, the latter also has to meet the requirements defined in the previous list of allergenic materials.

Analogously, in case the footwear comprises a reinforcement 5, the latter also has to meet the requirements defined in the previous list of allergenic materials.

The concentration limits defined in the list are computed as the ratio between the mass of the allergenic material found in a control volume of the shoe portion to be examined, wherein the shoe portion may be the upper, the insole, the lining or the reinforcement, and the mass of the same control volume of the shoe portion. In any case, the standards mentioned for each of the materials define the measurement procedure to be carry out for the assessment of the material concentrations.

The predefined list of allergenic materials and corresponding maximum concentrations, as referred to the upper 1, insole 2 and optionally the lining 4, may further include, in addition to the preceding material concentration requirements, at least one between:

- potassium dichromate, the potassium dichromate being present at most in a concentration lower than 0,01 mg/kg as Cr VI according to acid digestion and Inductively Coupled Plasma Emission Spectroscopy (ICP-OES) methods;

- cobalt chloride, the cobalt chloride being present at most in a concentration lower than 0,01 mg/kg as Co according to acid digestion and Inductively Coupled Plasma Emission Spectroscopy (ICP-OES) methods;

- nickel sulfate, the nickel sulfate being present at most in a concentration lower than 0,01 mg/kg as Ni according to acid digestion and Inductively Coupled Plasma Emission Spectroscopy (ICP-OES) methods.

Preferably, the maximum concentrations of all of the preceding allergenic materials, namely the potassium dichromate, the cobalt chloride and the nickel sulfate, have to satisfy simultaneously the corresponding concentration limits of 0,01 mg/kg.

According to an embodiment, the reinforcement 5 should also meet the requirements provided by this list of allergenic materials.

According to a further embodiment, the outsole 3 may also meet the requirements provided by this list of allergenic materials.

The predefined list of allergenic materials and corresponding maximum concentrations, as referred to the upper 1, insole 2 and optionally the lining 4, may further include, in addition to the preceding material concentration requirements, at least one between:

- paraphenylenediamine, the paraphenylenediamine being present at most in a concentration lower than 0,5 mg/kg according to solvent extraction and/or Gas Chromatography Mass Spectrometry (GC-MS) and/or Liquid Chromatography with UltraViolet LC-UV determination;
- an organotin compound, the organotin compound being present at most in a concentration lower than 0,02 mg/kg as Ni according to CEN ISO/TS 16179:2012;
- azo dyes, the azo dyes being present at most in a concentration lower than 5 mg/kg according to UNI EN 14362.

The reinforcement 5 of the shoe should also meet the above requirements in terms of maximum concentration of organotin compound, namely a concentration lower than 0,02 mg/kg.

The organotin compound may include one or more of the following substances and correlated maximum concentrations:

- Tributyltin (TBT) in a concentration lower than 0,02 mg/kg;

- Monobutyltin (MBT) in a concentration lower than 0,02 mg/kg;

- Monooctylzinn (MOT) in a concentration lower than 0,02 mg/kg;

- Dibutyltin (DBT) in a concentration lower than 0,02 mg/kg;

- Dibutyltin dichloride (DBTC) in a concentration lower than 0,02 mg/kg;

- Dioctyltin (DOT) in a concentration lower than 0,02 mg/kg;

- Triphenyltin (TPhT) in a concentration lower than 0,02 mg/kg;

- Bis (Tributyltin) oxide (TBTO) in a concentration lower than 0,02 mg/kg;

- Dipheyltin (DPhT) in a concentration lower than 0,02 mg/kg;

- Dipropyltin (DPT) in a concentration lower than 0,02 mg/kg;

- Monopheyltin (MPhT) in a concentration lower than 0,02 mg/kg;

- Tetrabutyltin (TeBT) in a concentration lower than 0,02 mg/kg;

- Tricyclohexyltin (TCyHT) in a concentration lower than 0,02 mg/kg;

- Trimethyltin (TMT) in a concentration lower than 0,02 mg/kg;

- Trioctyltin (TOT) in a concentration lower than 0,02 mg/kg;

- Tripropyltin (TPT) in a concentration lower than 0,02 mg/kg.

In particular, it is preferred that the footwear, in particular the upper 1 the insole 2 and the lining 4, of the present invention fulfills at least five, in particular at least ten or preferably all, the requirements specified in the above list of organotin compounds.

The upper, the lining and the insole of the shoe contact the user's foot during a use condition: therefore it is very important that these shoe portions meet the requirements, in terms of allergenic material concentrations, listed above.

Anyhow, also the outsole 3 may play a role in the ACD onset: thus, according to a further embodiment, the outsole 3 may also include a structure according to a predefine outsole list of allergenic materials and corresponding maximum concentrations as follows:

- chromium salts, said chromium salts being present at most in a concentration lower than 0,01 mg/kg according to DIN 54233-3:2010;

- dimethyl fumarate, said dimethyl fumarate being present at most in a concentration lower than 0,01 mg/kg according to EN ISO 16186:2012;

- Para tertiary butylphenol formaldehyde, said Para tertiary butylphenol formaldehyde being present at most in a concentration lower than 5 mg/kg according to EN ISO 14184-1:2011;

- formaldehyde, said formaldehyde being present at most in a concentration lower than 5 mg/kg according to EN ISO 14184-1:2011;

- chromium, soluble total, said chromium soluble total being present at most in a concentration lower than 0,01 mg/kg according to DIN 54233-3:2010.

Alternatively, the outsole may include a structure comprising the same predefined list of allergenic materials and corresponding maximum concentrations of the upper and the insole, in particular of the upper 1, insole 2 and of the lining 4. In this case, the predefined list should include:

- chromium salts, the chromium salts being present at most in a concentration lower than 0,01 mg/kg according to DIN 54233-3:2010;

- dimethyl fumarate, said dimethyl fumarate being present at most in a concentration lower than 0,01 mg/kg according to EN ISO 16186:2012;

- Para tertiary butylphenol formaldehyde, said Para tertiary butylphenol formaldehyde being present at most in a concentration lower than 5 mg/kg according to EN ISO 14184-1:2011;

- formaldehyde, said formaldehyde being present at most in a concentration lower than 5 mg/kg according to EN ISO 14184-1:2011;

- a chromium, soluble total, concentration lower than 0,01 mg/kg according to DIN 54233-3:2010;

- potassium dichromate, said potassium dichromate being present at most in a concentration lower than 0,01 mg/kg as Cr VI according to acid digestion and Inductively Coupled Plasma Emission Spectroscopy (ICP-OES) methods;

- cobalt chloride, said cobalt chloride being present at most in a concentration lower than 0,01 mg/kg as Co according to acid digestion and Inductively Coupled Plasma Emission Spectroscopy (ICP-OES) methods;

- nickel sulfate, said nickel sulfate being present at most in a concentration lower than 0,01 mg/kg as Ni according to acid digestion and Inductively Coupled Plasma Emission Spectroscopy (ICP-OES) methods.

- paraphenylenediamine, said paraphenylenediamine being present at most in a concentration lower than 0,5 mg/kg according to solvent extraction and/or Gas Chromatography Mass Spectrometry (GC-MS) and/or Liquid Chromatography with UltraViolet LC-UV determination;

- organotin compounds, said organotin compounds being present at most in a concentration lower than 0,02 mg/kg as Ni according to CEN ISO/TS 16179:2012;

- azo dyes, said azo dyes being present at most in a concentration lower than 5 mg/kg according to UNI EN 14362.

In particular, it is preferred that the outsole 3 fulfills at least 4, in particular at least eight or preferably all, the requirements specified in the above list of allergenic materials. For example, the outsole 3 may meet the requirements with respect to the corresponding concentration limits of the chromium salts, formaldehyde, potassium dichromate and azo dyes.

In addition, the predefined list of allergenic materials and maximum concentrations of the upper, in particular of the upper 1, the lining 4 and the insole 2, of the footwear may further include aromatic amines in a concentration lower than 5 mg/kg according to EN 14362-1:2017 or EN14362-2:2003/AC:2005 or EN 14362-3:2017.

The aromatic amines may comprise at least one substance between a:

- Benzidine concentration lower than 5 mg/kg;
- o-Anisidine concentration lower than 5 mg/kg;
- o-aminoazotoluene concentration lower than 5 mg/kg;
- o-toluidine concentration lower than 5 mg/kg;
- p-Cresidine concentration lower than 5 mg/kg;
- p-Chloroaniline concentration lower than 5 mg/kg;
- 2-Amino-4-Nitrotoluene concentration lower than 5 mg/kg;
- 2-Naphthylamine concentration lower than 5 mg/kg;
- 2,4-Toluylenediamine concentration lower than 5 mg/kg;
- 2,4 Xylidine concentration lower than 5 mg/kg;
- 2,6 Xylidine concentration lower than 5 mg/kg;
- 3,3' - Dimethylbenzidine concentration lower than 5 mg/kg;
- 3,3' - Dichlorobenzidine concentration lower than 5 mg/kg;
- 3,3' - Dimethoxybenzidine concentration lower than 5 mg/kg;
- 3,3' - Dimethyl - 4,4' - Diaminodiphenylmethane concentration lower than 5 mg/kg
- 4 - Aminodiphenyl concentration lower than 5 mg/kg;
- 4 - Chloro-O-Toluine concentration lower than 5 mg/kg;
- 4,4' - Diaminodiphenylmethane concentration lower than 5 mg/kg;
- 4,4' - Methylene-Bis-(2-Chloroaniline) concentration lower than 5 mg/kg;
- 4,4' - Thiodianiline concentration lower than 5 mg/kg;
- 4,4' - Oxydianiline concentration lower than 5 mg/kg;

- 2,4,5 - Trimethylaniline concentration lower than 5 mg/kg;
- 4 - Aminoazobenzene concentration lower than 5 mg/kg.

In particular the predefined list of allergenic material should include at least five or at least ten of the aromatic amine substances listed above. For example, the upper 1, the lining 4 and the insole 2 should fulfill the concentration requirements with respect to at least Benzidine, o-toluidine, Chloroaniline, Chloro-O-Toluine and Aminoazobenzene.

Optionally also the a collar and/or a reinforcement of the footwear may comprise aromatic amines in a concentration lower than 5 mg/kg according to EN 14362-1:2017 or EN14362-2:2003/AC:2005 or EN 14362-3:2017.

Furthermore, according to an embodiment, also the outsole 3 is made of a structure according to the list of aromatic amines and corresponding maximum concentrations.

In order to prevent ACD, the list of allergenic materials and corresponding maximum concentrations of the upper, in particular of the upper 1, lining 4 and insole 2, of the footwear further includes heavy metals, wherein the heavy metals are present at most in a concentration, in particular soluble heavy metal concentration, lowerthan 0,1 mg/kg according to DIN 54233-3:2010 and/or EN1122:2001 and/or acid digestion and/or Inductively Coupled Plasma Emission Spectroscopy (ICP-OES) method.

The heavy metals may include at least one substance between:

- Arsenic (As) in a concentration lower than 0,01 mg/kg;
- Cadmium (Cd) in a concentration lower than 0,01 mg/kg;
- Chromium in a concentration lower than 0,01 mg/kg;
- Cobalt (Co) in a concentration lower than 0,01 mg/kg;
- Lead (Pb) in a concentration lower than 0,01 mg/kg;
- Mercury (Hg) in a concentration lower than 0,01 mg/kg;
- Nickel (Ni) in a concentration lower than 0,01 mg/kg.

Optionally also a collar and/or the reinforcement of the footwear may comprise heavy metals in a concentration lower than 0,1 mg/kg according to DIN 54233-3:2010 and/or EN1122:2001 and/or acid digestion and/or Inductively Coupled Plasma Emission Spectroscopy (ICP-OES) method.

In general, the list of heavy metals and corresponding maximum concentrations may also comprise chemical elements having atomic number higher than 20, in particular higher than 23, or a density higher than 5 g/cm$^3$.

The predefined list of allergenic materials and corresponding maximum concentrations, which the footwear of the present invention should preferably adhere to, may further include:

- mercapto mix, said mercapto mix being present at most with a concentration lower than 0,1 mg/kg according to solvent extraction and determination in *HPLC-UV*;
- carba mix, said carba mix being present at most with a concentration lower than 0,1 mg/kg according to ...;
- Thiuram Mix, said Thiuram Mix being present at most with a concentration lower than 0,1 mg/kg;
- Mercaptobenziotaziolo, said Mercaptobenziotaziolo being present at most with a concentration lower than 0,1 mg/kg;
- Mixed Dialkil Thioureas, said Mixed Dialkil Thioureas being present at most with a concentration lower than 0,1 mg/kg;
- Black Rubber Mix, said Black Rubber Mix being present at most with a concentration lower than 0,1 mg/kg;
- Parabeni Mix, said Parabeni Mix being present at most with a concentration lower than 0,1 mg/kg.

In particular the mercapto mix may comprise at least one substance in the group including:

- N-Cyclohexylbenzothiazyl-sulfenamide
- cyclohexyl benzothiazolesulfenamide
- 2(cyclohexylaminothio)benzothiazole
- benzothiazyl-2-cyclohexylsulfenamide
- Dibenzothiazyl disulfide
- 2,2'-cithiobis(benzothiazole)
- dibenzothiazolyl disulfide
- 2,2'bis(benzothiazolyl) disulfide
- 2,2'-dibenzothiazyl disulfide
- 2-mercaptobenzothiazole disulfide
- Morpholinylmercaptobenzothiazole
- 2-benzothiazolyl morpholino disulfide
- 2morpholinodithiobenzothiazole
- benzothiazole 2-(4-morpholinyl)

- 4-morpholinyl 2-benzothiazyl disulfide
- Benzothiazole,2-(4-morpholinyldithio).

Furthermore, the carba mix may comprise at least one substance in the group including:

- Diphenylguanidine
- 1,3-Diphenylguanidine
- N,N'-Diphenylguanidine
- Zincdibutyldithiocarbamate
- bis (N,N-dibutyldithiocarbamato) zinc
- carbamic acid dibutyldithio-, zinc complex
- zinc bis (dibutyldithiocarbamate)
- Zincdiethyldithiocarbamate
- diethyldithiocarbamic acid zinc salt
- zinc bis (diethyldithiocarbamate)
- zinc diethylcarbamodithioate.

The footwear according to the present invention further defines a permeable structure having:

- a water vapour permeability equal to or higher than 2,0 mg/(cm$^2$·h), in particular higher than 1,3 mg/(cm$^2$·h), according to EN 13515:2001/ISO 17 699:2003; and/or
- a water vapour coefficient equal to or higher than 20 mg/cm$^2$, in particular higher than 50 mg/cm$^2$, according to EN 13515:2001/ISO 17 699:2003.

The disclosed and claimed limits relative to the water vapour permeability and the water vapour coefficient are determined in order to prevent or reduce the risk of ACD onset, in combination with the requirements relative to the concentration limits of allergenic materials included in the predefined list or lists described above. In particular, the water vapour permeability and the water vapour coefficient values, respectively higher or equal to 2,0 mg/(cm$^2$·h) and 20 mg/cm$^2$, are according to any of the concentration limits disclosed in the above lists of allergenic materials.

The water vapour permeability and the water vapour coefficient values defined above determines a sufficient perspiration to reduce moisture and/or the maximum temperature within the footwear in a use condition: indeed, high moisture and high temperature values within the footwear increase the risk of ACD onset. Notably, lack of perspiration of the footwear leads to temperature increase, causing thereby sweating of the user's foot, which in turn increases the risk of ACD onset. Furthermore, the combination of high temperature, lack of perspiration and the presence of the allergenic materials of the predefined list over the concentration limits determines a further increment in the risk of ACD onset. Therefore, limiting the internal temperature of the footwear by imposing a sufficient perspiration, defined by the water vapour permeability and the water vapour coefficient, and, at the same time, limiting the presence of allergenic materials under predetermined concentration limits, leads to a synergic effect of considerably reducing the risk of ACD onset.

In addition, it is preferable that the permeable structure has a water vapour absorption equal to or higher than 1 mg/cm$^2$, in particular higher than 1,5 mg/cm$^2$, according to EN 13515:2001/ISO 17 699:2003.

In addition, the footwear of the present invention has a dermocompatibility defined by a growth inhibition percentage equal to or lower than 30%, in particular lower than 25%, according to DIN EN ISO 10993, DIN EN ISO 10993-5.

In particular, at least the upper 1 and the insole 2 have a dermocompatibility defined by a growth inhibition percentage equal to or lower than 30%, in particular lower than 25%, according to DIN EN ISO 10993, DIN EN ISO 10993-5.

In the case the footwear 10 includes a lining 4, at least the lining 4 and the insole 2 have a dermocompatibility defined by a growth inhibition percentage equal to or lower than 30%, in particular lower than 25%, according to DIN EN ISO 10993, DIN EN ISO 10993-5.

Optionally, in case the footwear 10 includes the lining 4, at least said lining 4, the upper 1 and the insole 2 have a dermocompatibility defined by a growth inhibition percentage equal to or lower than 30%, in particular lower than 25%, according to DIN EN ISO 10993, DIN EN ISO 10993-5.

The dermocompatibility defined by the growth inhibition percentage is computed by the following formula:

$$\%WH = 100 - 100 \cdot \frac{(OD_{570nm}sample) - (OD_{570nm}blank)}{(OD_{570nm}control) - (OD_{570nm}blank)}$$

wherein:

$OD_{570nm}$ = optical density quantified at 570 nm wavelengths;

$OD_{570nm}$ Sample = Average extinction of a sample dilution out of 3 parallel wells;

$OD_{570nm}$ Blank = Average extinction of a blank dilution out of 3 parallel wells, the blank dilution being without cells;

$OD_{570nm}$ Control = Average extinction of the solvent control out of 3 parallel wells.

The DIN EN ISO 10993, DIN EN ISO 10993-5. 2009-10A provides all the technical aspects to allow a skilled person to compute the growth inhibition percentage by tests for in vitro cytotoxicity.

Furthermore, the footwear may also has a pH value comprised between 5 and 7,5 according to EN ISO 3071 :2020, in particular a pH value comprised between 5,5 and 7. In particular the insole 2 preferably has a pH value falling within the above mentioned pH interval. Analogously, it is preferable that the upper 1 has a pH value falling within the above mentioned pH interval. Still, also the lining 4 may have a pH value falling within the above mentioned pH interval. In a preferred embodiment, the upper, the lining and the insole have a pH value falling within the above mentioned pH interval, namely between 5 and 7,5, more in particular between 5,5 and 7.

Accordingly, also the collar and a reinforcement of the footwear may have a pH value comprised between 5 and 8 according to EN ISO 3071 :2020, in particular a pH value comprised between 5,5 and 7.

The footwear also has to be resistant to support the mechanical stresses which it undergoes during use conditions. According to this requirement, the upper has an ultimate strength higher than or equal to 500 N/50mm according to UNI EN ISO 13934-1:2013, in particular higher than 1000 N/50mm.

The footwear should also fulfill requirements with respect to an adequate elongation before failure in a strength test: thus, it may be required that the upper ad an ultimate elongation at failure higher than or equal to 15% according to UNI EN ISO 13934-1:2013, in particular higher than 40%, more in particular higher than 55%.

The footwear may be mainly composed by nylon and/or polyester or a combination thereof, wherein the nylon and/or polyester are present at least in a percentage higher than 50%, in particular higher than 75%, more in particular higher than 85%.

In more detail, the upper is mainly composed by nylon and/or polyester or a combination thereof, wherein the nylon and/or polyester are present at least in a percentage higher than 70%, in particular higher than 85%, more in particular higher than 95%.

Furthermore, the insole may also be mainly composed by nylon and/or polyester or a combination thereof, wherein the nylon and/or polyester are present at least in a percentage higher than 70%, in particular higher than 85%, more in particular higher than 95%.

Analogously, the lining may be mainly composed by nylon and/or polyester or a combination thereof, wherein the nylon and/or polyester are present at least in a percentage higher than 70%, in particular higher than 85%, more in particular higher than 95%.

Notably, although these materials, namely nylon and polyester, may already fulfill the requirements in terms of hypo-allergenicity and dermocompatibility, it must be considered that the fabrication methods, and the material manufacturing as well, may cause growth of undesired allergenic substances, which may negatively affect the allergenicity and dermocompatibility values. In other terms, the fact that a footwear is mainly made by nylon and polyester may be not sufficient to satisfy the requirements of the footwear of the present invention.

**Method to prevent onset of ACD**

The present invention also relates to a method to prevent onset of allergic contact dermatitis ACD in a user's foot by wearing the hypoallergenic footwear described above and according to the appended claims.

The method also relates the use of the hypoallergenic footwear described above and according to the appended claims in order to prevent onset of allergic contact dermatitis ACD.

**Claims**

1. Method for preventing allergic contact dermatitis (ACD) in a user's foot by wearing a hypoallergenic footwear (10), wherein said footwear comprises at least an upper (1), an insole (2) and an outsole (3),

   - wherein at least the upper (1) and the insole (2) have hypoallergenic structure according to a predefined list of allergenic materials and corresponding maximum concentrations, said list comprising:

     ◦ chromium salt, the chromium salt being present at most in a concentration lower than 0,01 mg/kg according to DIN 54233-3:2010;
  ◦ dimethyl fumarate, the dimethyl fumarate being present at most in a concentration lower than 0,01 mg/kg according to EN ISO 16186:2012;
  ◦ para tertiary butylphenol formaldehyde, the para tertiary butylphenol formaldehyde being present at most in a concentration lower than 5 mg/kg according to EN ISO 14184-1:2011;
  ◦ formaldehyde, the formaldehyde being present at most in a concentration lower than 5 mg/kg according to EN ISO 14184-1:2011;
  ◦ chromium, soluble total, the chromium being present at most in a concentration lower than 0,01 mg/kg according to DIN 54233-3:2010;

- wherein said footwear (10), in particular at least the upper (1), defines a permeable structure having:

  ◦ a water vapour permeability equal to or higher than 2,0 mg/(cm$^2$·h), in particular higher than 1,3 mg/(cm$^2$·h), according to EN 13515:2001/ISO 17 699:2003; and/or
  ◦ a water vapour coefficient equal to or higher than 20 mg/cm$^2$, in particular higher than 50 mg/cm$^2$, according to EN 13515:2001/ISO 17 699:2003;

- and wherein at least the insole (2) has, optionally at least the upper (1) and the insole (2) have, a dermocompatibility defined by a growth inhibition percentage equal to or lower than 30%, in particular lower than 25%, according to DIN EN ISO 10993, DIN EN ISO 10993-5.

2. Hypoallergenic footwear (10) comprising at least an upper (1), an insole (2) and an outsole (3),

- wherein at least the upper (1) and the insole (2) have hypoallergenic structure according to a predefined list of allergenic materials and corresponding maximum concentrations, said list comprising:

  ◦ chromium salt, the chromium salt being present at most in a concentration lower than 0,01 mg/kg according to DIN 54233-3:2010;
  ◦ dimethyl fumarate, the dimethyl fumarate being present at most in a concentration lower than 0,01 mg/kg according to EN ISO 16186:2012;
  ◦ para tertiary butylphenol formaldehyde, the para tertiary butylphenol formaldehyde being present at most in a concentration lower than 5mg/kg according to EN ISO 14184-1:2011;
  ◦ formaldehyde, the formaldehyde being present at most in a concentration lower than 5 mg/kg according to EN ISO 14184-1:2011;
  ◦ chromium, soluble total, the chromium being present at most in a concentration lower than 0,01 mg/kg according to DIN 54233-3:2010;

- wherein said footwear (10), in particular at least the upper (1), defines a permeable structure having:

  ◦ a water vapour permeability equal to or higher than 2,0 mg/(cm$^2$·h), in particular higher than 1,3 mg/(cm$^2$·h), according to EN 13515:2001/ISO 17 699:2003; and/or
  ◦ a water vapour coefficient equal to or higher than 20 mg/cm$^2$, in particular higher than 50 mg/cm$^2$, according to EN 13515:2001/ISO 17 699:2003;

- and wherein at least the insole (2) has, optionally at least the upper (1) and the insole (2) have, a dermocompatibility defined by a growth inhibition percentage equal to or lower than 30%, in particular lower than 25%, according to DIN EN ISO 10993, DIN EN ISO 10993-5.

3. The method or the footwear of any one of the preceding claims, wherein the footwear (10) further includes a lining (4) comprising the hypoallergenic structure according to said list of predefined maximum concentrations of allergenic materials, said lining (4) contacting the user's foot when the hypoallergenic footwear (10) is in use, wherein the lining (4) has a dermocompatibility defined by a growth inhibition percentage equal to or lower than 30%, in particular lower than 25%, according to DIN EN ISO 10993, DIN EN ISO 10993-5.

4. The method or the footwear of any one of the preceding claims, wherein said predefined list of allergenic materials and corresponding maximum concentrations further includes:

  ◦ potassium dichromate, the potassium dichromate being present at most in a concentration lower than 0,01 mg/kg as Cr VI according to acid digestion and Inductively Coupled Plasma Emission Spectroscopy (ICP-OES) methods;
◦ cobalt chloride, the cobalt chloride being present at most in a concentration lower than 0,01 mg/kg as Co according to acid digestion and Inductively Coupled Plasma Emission Spectroscopy (ICP-OES) methods;
◦ nickel sulfate, the nickel sulfate being present at most in a concentration lower than 0,01 mg/kg as Ni according to acid digestion and Inductively Coupled Plasma Emission Spectroscopy (ICP-OES) methods.

5. The method or the footwear of any one of the preceding claims, wherein said list of allergenic materials of the upper (1) and the insole (2), optionally of the lining (4), insole (2) and preferably also of the upper (1), further includes:

◦ paraphenylenediamine, the paraphenylenediamine being present at most in a concentration lower than 0,5 mg/kg according to solvent extraction and/or Gas Chromatography Mass Spectrometry (GC-MS) and/or Liquid Chromatography with UltraViolet (LC-UV) determination;
◦ an organotin compound, the organotin compound being present at most in a concentration lower than 0,02 mg/kg as Ni according to CEN ISO/TS 16179:2012;
◦ azo dyes, the azo dyes being present at most in a concentration lower than 5 mg/kg according to UNI EN 14362.

6. The method or the footwear of any one of the preceding claims, wherein the outsole (3) has a dermocompatibility higher than 30% and:

- a structure comprising the same predefined list of allergenic materials and corresponding maximum concentrations of the upper (1) and the insole (2), in particular of the upper (1), insole (2) and of the lining (4), in particular said list of allergenic materials comprising all or at least one substance between:

◦ chromium salts, the chromium salts being present at most in a concentration lower than 0,01 mg/kg according to DIN 54233-3:2010;
◦ dimethyl fumarate, said dimethyl fumarate being present at most in a concentration lower than 0,01 mg/kg according to EN ISO 16186:2012;
◦ Para tertiary butylphenol formaldehyde, said Para tertiary butylphenol formaldehyde being present at most in a concentration lower than 5mg/kg according to EN ISO 14184-1:2011;
◦ formaldehyde, said formaldehyde being present at most in a concentration lower than 5 mg/kg according to EN ISO 14184-1:2011;
◦ a chromium, soluble total, concentration lower than 0,01 mg/kg according to DIN 54233-3:2010;
◦ potassium dichromate, said potassium dichromate being present at most in a concentration lower than 0,01 mg/kg as Cr VI according to acid digestion and Inductively Coupled Plasma Emission Spectroscopy (ICP-OES) methods;
◦ cobalt chloride, said cobalt chloride being present at most in a concentration lower than 0,01 mg/kg as Co according to acid digestion and Inductively Coupled Plasma Emission Spectroscopy (ICP-OES) methods;
◦ nickel sulfate, said nickel sulfate being present at most in a concentration lower than 0,01 mg/kg as Ni according to acid digestion and Inductively Coupled Plasma Emission Spectroscopy (ICP-OES) methods.
◦ paraphenylenediamine, said paraphenylenediamine being present at most in a concentration lower than 0,5 mg/kg according to solvent extraction and/or Gas Chromatography Mass Spectrometry (GC-MS) and/or Liquid Chromatography with UltraViolet (LC-UV) determination;
◦ organotin compounds, said organotin compounds being present at most in a concentration lower than 0,02 mg/kg as Ni according to CEN ISO/TS 16179:2012;
◦ azo dyes, said azo dyes being present at most in a concentration lower than 5 mg/kg according to UNI EN 14362;

or
- a second distinct list of allergenic materials including all or at least one substance between:

◦ chromium salts, said chromium salts being present at most in a concentration lower than 0,01 mg/kg according to DIN 54233-3:2010;
◦ dimethyl fumarate, said dimethyl fumarate being present at most in a concentration lower than 0,01 mg/kg according to EN ISO 16186:2012;
◦ Para tertiary butylphenol formaldehyde, said Para tertiary butylphenol formaldehyde being present at most in a concentration lower than 5 mg/kg according to EN ISO 14184-1:2011;

∘ formaldehyde, said formaldehyde being present at most in a concentration lower than 5 mg/kg according to EN ISO 14184-1:2011;
∘ chromium, soluble total, said chromium soluble total being present at most in a concentration lower than 0,01 mg/kg according to DIN 54233-3:2010.

7. The method or the footwear of the preceding claim 6, wherein said second distinct list of allergenic materials further includes:

- a potassium dichromate concentration lower than 0,01 mg/kg as Cr VI according to acid digestion and Inductively Coupled Plasma Emission Spectroscopy (ICP-OES) methods;
- a cobalt chloride concentration lower than 0,01 mg/kg as Co according to acid digestion and Inductively Coupled Plasma Emission Spectroscopy (ICP-OES) methods;
- a nickel sulfate concentration lower than 0,01 mg/kg as Ni according to acid digestion and Inductively Coupled Plasma Emission Spectroscopy (ICP-OES) methods;
- a paraphenylenediamine concentration lower than 0,5 mg/kg according to solvent extraction and/or Gas Chromatography Mass Spectrometry (GC-MS) and/or Liquid Chromatography with UltraViolet LC-UV determination;
- an organotin compounds concentration lower than 0,02 mg/kg as Ni according to CEN ISO/TS 16179:2012;
- an azo dyes concentration lower than 5 mg/kg according to UNI EN 14362.

8. The method or the footwear of any one of the preceding claims, wherein the predefined list of allergenic materials and corresponding maximum concentrations of the upper (1), in particular of the upper (1), lining (4) and insole (2), of the footwear (10) further includes:

- aromatic amines, said aromatic amines being present at most in a concentration lower than 5 mg/kg according to EN 14362-1:2017 or EN14362-2:2003/AC:2005 or EN 14362-3:2017, wherein the aromatic amines comprise one or more of:

∘ Benzidine, said benzidine being present at most with a concentration lower than 5 mg/kg;
∘ o-Anisidine, said o-Anisidine being present at most with a concentration lower than 5 mg/kg;
∘ o-aminoazotoluene, said o-aminoazotoluene being present at most with a concentration lower than 5 mg/kg;
∘ o-toluidine, said o-toluidine being present at most with a concentration lower than 5 mg/kg;
∘ p-Cresidine, said p-Cresidine being present at most with a concentration lower than 5 mg/kg;
∘ p-Chloroaniline, said Chloroaniline being present at most with a concentration lower than 5 mg/kg;
∘ 2-Amino-4-Nitrotoluene, said 2-Amino-4-Nitrotoluene being present at most with a concentration lower than 5 mg/kg;
∘ 2-Naphthylamine, said 2-Naphthylamine being present at most with a concentration lower than 5 mg/kg;
∘ 2,4-Toluylenediamine, said 2,4-Toluylenediamine being present at most with a concentration lower than 5 mg/kg;
∘ 2,4 Xylidine, said 2,4 Xylidine being present at most with a concentration lower than 5 mg/kg;
∘ 2,6 Xylidine, said 2,6 Xylidine being present at most with a concentration lower than 5 mg/kg;
∘ 3,3' - Dimethylbenzidine, said 3,3' Dimethylbenzidine being present at most with a concentration lower than 5 mg/kg;
∘ 3,3' - Dichlorobenzidine, said 3,3' Dichlorobenzidine being present at most with a concentration lower than 5 mg/kg;
∘ 3,3' - Dimethoxybenzidine, said 3,3' Dimethoxybenzidine being present at most with a concentration lower than 5 mg/kg;
∘ 3,3' - Dimethyl - 4,4' - Diaminodiphenylmethane, said 3' - Dimethyl - 4,4' - Diaminodiphenylmethane being present at most with a concentration lower than 5 mg/kg;
∘ 4 - Aminodiphenyl, said 4 - Aminodiphenyl being present at most with a concentration lower than 5 mg/kg;
∘ 4 - Chloro-O-Toluine, said 4 - Chloro-O-Toluine being present at most with a concentration lower than 5 mg/kg;
∘ 4,4' - Diaminodiphenylmethane, said 4,4' - Diaminodiphenylmethane being present at most with a concentration lower than 5 mg/kg;
∘ 4,4' - Methylene-Bis-(2-Chloroaniline), said 4,4' - Methylene-Bis-(2-Chloroaniline) being present at most with a concentration lower than 5 mg/kg;
∘ 4,4' - Thiodianiline, said Thiodianiline being present at most with a concentration lower than 5 mg/kg;
∘ 4,4' - Oxydianiline, said 4,4' - Oxydianiline being present at most with a concentration lower than 5 mg/kg;

○ 2,4,5 - Trimethylaniline, said 2,4,5 - Trimethylaniline being present at most with a concentration lower than 5 mg/kg;

○ 4 - Aminoazobenzene, said 4 - Aminoazobenzene being present at most with a concentration lower than 5 mg/kg;

- heavy metal(s), said heavy metal(s) being present at most in concentration, in particular soluble heavy metal concentration, lower than 0,1 mg/kg according to DIN 54233-3:2010 and/or EN1122:2001 and/or acid digestion and/or Inductively Coupled Plasma Emission Spectroscopy (ICP-OES) method, optionally said heavy metals include at least one between a:

○ Arsenic (As) concentration lower than 0,01 mg/kg;
○ Cadmium (Cd) concentration lower than 0,01 mg/kg;
○ Chromium concentration lower than 0,01 mg/kg;
○ Cobalt (Co) concentration lower than 0,01 mg/kg;
○ Lead (Pb) concentration lower than 0,01 mg/kg;
○ Mercury (Hg) concentration lower than 0,01 mg/kg;
○ Nickel (Ni) concentration lower than 0,01 mg/kg;

optionally the footwear (10) further including a collar (6) and/or a reinforcement (5) comprising said aromatic amines in a concentration lower than 5 mg/kg according to EN 14362-1 :2017 or EN14362-2:2003/AC:2005 or EN 14362-3:2017, and/or said heavy metal(s) in a concentration lower than 0,1 mg/kg according to DIN 54233-3:2010 and/or EN1122:2001 and/or acid digestion and/or Inductively Coupled Plasma Emission Spectroscopy (ICP-OES) method.

9. The method or the footwear of any one of the preceding claims, wherein at least one between the upper (1), the lining (4) and the insole (2) of the footwear (10) has a pH value comprised between 5 and 7,5 according to EN ISO 3071 :2020, in particular a pH value comprised between 5,5 and 7.

10. The method or the footwear of any one of the preceding claims, wherein the list of allergenic materials and corresponding concentrations and/or the second distinct list of allergenic materials include:

- mercapto mix, said mercapto mix being present at most with a concentration lower than 0,1 mg/kg according to solvent extraction and determination in *HPLC-UV*;
- carba mix, said carba mix being present at most with a concentration lower than 0,1 mg/kg according to ...;
- Thiuram Mix, said Thiuram Mix being present at most with a concentration lower than 0,1 mg/kg;
- Mercaptobenziotaziolo, said Mercaptobenziotaziolo being present at most with a concentration lower than 0,1 mg/kg;
- Mixed Dialkil Thioureas, said Mixed Dialkil Thioureas being present at most with a concentration lower than 0,1 mg/kg;
- Black Rubber Mix, said Black Rubber Mix being present at most with a concentration lower than 0,1 mg/kg;
- Parabeni Mix, said Parabeni Mix being present at most with a concentration lower than 0,1 mg/kg.

11. The method or the footwear of any one of the preceding claims, wherein the footwear (10) further comprises one or more of:

- a reinforcement (5) according to said predefined list of allergenic materials and corresponding maximum concentrations;
- a collar (6) according to said predefined list of allergenic materials and corresponding maximum concentrations.

12. The method or the footwear according to any one of the preceding claims, wherein the permeable structure has a water vapour absorption equal to or higher than 1 mg/cm$^2$, in particular higher than 1,5 mg/cm$^2$, according to EN 13515:2001/ISO 17 699:2003.

13. The method or the footwear according to any one of the preceding claims, wherein the upper has:

- an ultimate strength higher than or equal to 500 N/50mm according to UNI EN ISO 13934-1 :2013, in particular higher than 1000 N/50mm; and/or
- an ultimate elongation at failure higher than or equal to 15% according to UNI EN ISO 13934-1:2013, in particular higher than 40%, more in particular higher than 55%.

14. The method or the footwear according to any one of the preceding claims, wherein the dermocompatibility defined by growth inhibition percentage is computed by the following formula:

$$\%WH = 100 - 100 \cdot \frac{(OD_{570nm}sample) - (OD_{570nm}blank)}{(OD_{570nm}control) - (OD_{570nm}blank)}$$

wherein:

$OD_{570nm}$ = optical density quantified at 570 nm wavelengths;
$OD_{570nm}$ Sample = Average extinction of a sample dilution out of 3 parallel wells;
$OD_{570nm}$ Blank = Average extinction of a blank dilution out of 3 parallel wells, the blank dilution being without cells;
$OD_{570nm}$ Control = Average extinction of the solvent control out of 3 parallel wells.

15. The method or the footwear according to any one of the preceding claims, wherein the upper (1), in particular the upper (1) and the lining (4), are based on, and particularly made of, nylon or polyester or bio natural fabrics, such as organic cotton and hemp, or a combination thereof and does/do not comprise animal-derived materials.

**Patentansprüche**

1. Verfahren zum Verhindern einer allergischen Kontaktdermatitis (ACD) an einem Fuß eines Benutzers durch Tragen eines hypoallergenen Schuhwerks (10), wobei das Schuhwerk wenigstens eine Oberschale (1), eine Innensohle (2) und eine Außensohle (3) umfasst,

- wobei wenigstens die Oberschale (1) und die Innensohle (2) gemäß einer vordefinierten Liste mit allergenen Materialien und entsprechenden Maximalkonzentrationen eine hypoallergene Struktur aufweisen, wobei die Liste umfasst:

- Chromsalz, wobei das Chromsalz gemäß DIN 54233-3:2010 höchstens in einer Konzentration von weniger als 0,01 mg/kg vorhanden ist;
- Dimethylfumarat, wobei das Dimethylfumarat gemäß EN ISO 16186:2012 höchstens in einer Konzentration von weniger als 0,01 mg/kg vorhanden ist;
- para-tertiäres Butylphenolformaldehyd, wobei das para-tertiäre Butylphenolformaldehyd gemäß EN ISO 14184-1:2011 höchstens in einer Konzentration von weniger als 5 mg/kg vorhanden ist;
- Formaldehyd, wobei das Formaldehyd gemäß EN ISO 14184-1:2011 höchstens in einer Konzentration von weniger als 5 mg/kg vorhanden ist;
- Chrom, vollständig lösbar, wobei das Chrom gemäß DIN 54233-3:2010 höchstens in einer Konzentration von weniger als 0,01 mg/kg vorhanden ist;

- wobei das Schuhwerk (10), insbesondere wenigstens die Oberschale (1), eine durchlässige Struktur definiert, aufweisend:

- eine Wasserdampfdurchlässigkeit, welche gemäß EN 13515:2001/ISO 17 699:2003 gleich wie oder größer als 2,0 mg/(cm$^2$·h) ist, insbesondere größer als 1,3 mg/(cm$^2$·h) ist; und/oder
- einen Wasserdampfkoeffizienten, welcher gemäß EN 13515:2001/ISO 17 699:2003 gleich wie oder größer als 20 mg/cm$^2$ ist, insbesondere größer als 50 mg/cm$^2$ ist;

- und wobei wenigstens die Innensohle (2), optional wenigstens die Oberschale (1) und die Innensohle (2), eine Hautverträglichkeit aufweist/aufweisen, welche durch einen Wachstumshemmungsprozentsatz definiert ist, welcher gemäß DIN EN ISO 10993, DIN EN ISO 10993-5 gleich wie oder weniger als 30% ist, insbesondere weniger als 25% ist.

2. Hypoallergenes Schuhwerk (10), umfassend wenigstens eine Oberschale (1), eine Innensohle (2) und eine Außensohle (3),

- wobei wenigstens die Oberschale (1) und die Innensohle (2) gemäß einer vordefinierten Liste mit allergenen Materialien und entsprechenden Maximalkonzentrationen eine hypoallergene Struktur aufweisen, wobei die Liste umfasst:

- Chromsalz, wobei das Chromsalz gemäß DIN 54233-3:2010 höchstens in einer Konzentration von weniger als 0,01 mg/kg vorhanden ist;
- Dimethylfumarat, wobei das Dimethylfumarat gemäß EN ISO 16186:2012 höchstens in einer Konzentration von weniger als 0,01 mg/kg vorhanden ist;
- para-tertiäres Butylphenolformaldehyd, wobei das para-tertiäre Butylphenolformaldehyd gemäß EN ISO 14184-1:2011 höchstens in einer Konzentration von weniger als 5 mg/kg vorhanden ist;
- Formaldehyd, wobei das Formaldehyd gemäß EN ISO 14184-1:2011 höchstens in einer Konzentration von weniger als 5 mg/kg vorhanden ist;
- Chrom, vollständig lösbar, wobei das Chrom gemäß DIN 54233-3:2010 höchstens in einer Konzentration von weniger als 0,01 mg/kg vorhanden ist;

- wobei das Schuhwerk (10), insbesondere wenigstens die Oberschale (1), eine durchlässige Struktur definiert, aufweisend:

- eine Wasserdampfdurchlässigkeit, welche gemäß EN 13515:2001/ISO 17 699:2003 gleich wie oder größer als 2,0 mg/(cm$^2$·h) ist, insbesondere größer als 1,3 mg/(cm$^2$·h) ist; und/oder
- einen Wasserdampfkoeffizienten, welcher gemäß EN 13515:2001/ISO 17 699:2003 gleich wie oder größer als 20 mg/cm$^2$ ist, insbesondere größer als 50 mg/cm$^2$ ist;

- und wobei wenigstens die Innensohle (2), optional wenigstens die Oberschale (1) und die Innensohle (2), eine Hautverträglichkeit aufweist/aufweisen, welche durch einen Wachstumshemmungsprozentsatz definiert ist, welcher gemäß DIN EN ISO 10993, DIN EN ISO 10993-5 gleich wie oder weniger als 30% ist, insbesondere weniger als 25% ist.

3. Verfahren oder Schuhwerk nach einem der vorhergehenden Ansprüche, wobei das Schuhwerk (10) ferner eine Fütterung (4) umfasst, welche die hypoallergene Struktur gemäß der Liste mit vordefinierten Maximalkonzentrationen allergener Materialien umfasst, wobei die Fütterung (4) den Fuß des Benutzers kontaktiert, wenn das hypoallergene Schuhwerk (10) verwendet wird,
wobei die Fütterung (4) eine Hautverträglichkeit aufweist, welche durch einen Wachstumshemmungsprozentsatz definiert ist, welcher gemäß DIN EN ISO 10993, DIN EN ISO 10993-5 gleich wie oder weniger als 30% ist, insbesondere weniger als 25% ist.

4. Verfahren oder Schuhwerk nach einem der vorhergehenden Ansprüche, wobei die vordefinierte Liste mit allergenen Materialien und entsprechenden Maximalkonzentrationen ferner umfasst:

- Kaliumdichromat, wobei das Kaliumdichromat gemäß Säureaufschluss und Emissionsspektroskopie mit induktiv gekoppeltem Plasma-(ICP-OES)-Verfahren höchstens in einer Konzentration von weniger als 0,01 mg/kg als Cr VI vorhanden ist;
- Kobaltchlorid, wobei das Kobaltchlorid gemäß Säureaufschluss und Emissionsspektroskopie mit induktiv gekoppeltem Plasma-(ICP-OES)-Verfahren höchstens in einer Konzentration von weniger als 0,01 mg/kg als Co vorhanden ist;
- Nickelsulfat, wobei das Nickelsulfat gemäß Säureaufschluss und Emissionsspektroskopie mit induktiv gekoppeltem Plasma-(ICP-OES)-Verfahren höchstens in einer Konzentration von weniger als 0,01 mg/kg als Ni vorhanden ist;

5. Verfahren oder Schuhwerk nach einem der vorhergehenden Ansprüche, wobei die Liste mit allergenen Materialien der Oberschale (1) und der Innensohle (2), optional der Fütterung (4), der Innensohle (2) sowie vorzugsweise ebenfalls der Oberschale (1), ferner umfasst:

- Paraphenylendiamin, wobei das Paraphenylendiamin gemäß Lösungsmittelextraktion und/oder Gaschromatographie-Massenspektrometrie (GC-MS) und/oder Flüssigkeitschromatographie mit Ultraviolett-(LC-UV)-Bestimmung höchstens in einer Konzentration von weniger als 0,5 mg/kg vorhanden ist;
- eine Organozinnverbindung, wobei die Organozinnverbindung gemäß CEN ISO/TS 16179:2012 höchstens in einer Konzentration von weniger als 0,02 mg/kg als Ni vorhanden ist;

- Azofarbstoffe, wobei die Azofarbstoffe gemäß UNI EN 14362 höchstens in einer Konzentration von weniger als 5 mg/kg vorhanden sind.

6. Verfahren oder Schuhwerk nach einem der vorhergehenden Ansprüche, wobei die Außensohle (3) eine Hautverträglichkeit von mehr als 30% aufweist sowie:

- eine Struktur, umfassend die gleiche vordefinierte Liste mit allergenen Materialien und entsprechenden Maximalkonzentrationen der Oberschale (1) und der Innensohle (2), insbesondere der Oberschale (1), der Innensohle (2) und der Fütterung (4), insbesondere wobei die Liste mit allergenen Materialien alle oder wenigstens eine Substanz umfasst aus:

- Chromsalzen, wobei die Chromsalze gemäß DIN 54233-3:2010 höchstens in einer Konzentration von weniger als 0,01 mg/kg vorhanden sind;
- Dimethylfumarat, wobei das Dimethylfumarat gemäß EN ISO 16186:2012 höchstens in einer Konzentration von weniger als 0,01 mg/kg vorhanden ist;
- para-tertiärem Butylphenolformaldehyd, wobei das para-tertiäre Butylphenolformaldehyd gemäß EN ISO 14184-1:2011 höchstens in einer Konzentration von weniger als 5 mg/kg vorhanden ist;
- Formaldehyd, wobei das Formaldehyd gemäß EN ISO 14184-1:2011 höchstens in einer Konzentration von weniger als 5 mg/kg vorhanden ist;
- Chrom, vollständig lösbar, gemäß DIN 54233-3:2010 mit einer Konzentration von weniger als 0,01 mg/kg;
- Kaliumdichromat, wobei das Kaliumdichromat gemäß Säureaufschluss und Emissionsspektroskopie mit induktiv gekoppeltem Plasma-(ICP-OES)-Verfahren höchstens in einer Konzentration von weniger als 0,01 mg/kg als Cr VI vorhanden ist;
- Kobaltchlorid, wobei das Kobaltchlorid gemäß Säureaufschluss und Emissionsspektroskopie mit induktiv gekoppeltem Plasma-(ICP-OES)-Verfahren höchstens in einer Konzentration von weniger als 0,01 mg/kg als Co vorhanden ist;
- Nickelsulfat, wobei das Nickelsulfat gemäß Säureaufschluss und Emissionsspektroskopie mit induktiv gekoppeltem Plasma-(ICP-OES)-Verfahren höchstens in einer Konzentration von weniger als 0,01 mg/kg als Ni vorhanden ist;
- Paraphenylendiamin, wobei das Paraphenylendiamin gemäß Lösungsmittelextraktion und/oder Gaschromatographie-Massenspektrometrie (GC-MS) und/oder Flüssigkeitschromatographie mit Ultraviolett-(LC-UV)-Bestimmung höchstens in einer Konzentration von weniger als 0,5 mg/kg vorhanden ist;
- Organozinnverbindungen, wobei die Organozinnverbindungen gemäß CEN ISO/TS 16179:2012 höchstens in einer Konzentration von weniger als 0,02 mg/kg als Ni vorhanden sind;
- Azofarbstoffe, wobei die Azofarbstoffe gemäß UNI EN 14362 höchstens in einer Konzentration von weniger als 5 mg/kg vorhanden sind;

oder
eine zweite verschiedene Liste mit allergenen Materialien, welche alle oder wenigstens eine Substanz umfasst aus:

- Chromsalzen, wobei die Chromsalze gemäß DIN 54233-3:2010 höchstens in einer Konzentration von weniger als 0,01 mg/kg vorhanden sind;
- Dimethylfumarat, wobei das Dimethylfumarat gemäß EN ISO 16186:2012 höchstens in einer Konzentration von weniger als 0,01 mg/kg vorhanden ist;
- para-tertiärem Butylphenolformaldehyd, wobei das para-tertiäre Butylphenolformaldehyd gemäß EN ISO 14184-1:2011 höchstens in einer Konzentration von weniger als 5 mg/kg vorhanden ist;
- Formaldehyd, wobei das Formaldehyd gemäß EN ISO 14184-1:2011 höchstens in einer Konzentration von weniger als 5 mg/kg vorhanden ist;
- Chrom, vollständig lösbar, wobei das vollständig lösbare Chrom gemäß DIN 54233-3:2010 höchstens in einer Konzentration von weniger als 0,01 mg/kg vorhanden ist.

7. Verfahren oder Schuhwerk nach Anspruch 6, wobei die zweite verschiedene Liste mit allergenen Materialien ferner umfasst:

- eine Kaliumdichromatkonzentration, welche gemäß Säureaufschluss und Emissionsspektroskopie mit induktiv gekoppeltem Plasma-(ICP-OES)-Verfahren weniger als 0,01 mg/kg als Cr VI beträgt;
- eine Kobaltchloridkonzentration, welche gemäß Säureaufschluss und Emissionsspektroskopie mit induktiv gekoppeltem Plasma-(ICP-OES)-Verfahren weniger als 0,01 mg/kg als Co beträgt;

- eine Nickelsulfatkonzentration, welche gemäß Säureaufschluss und Emissionsspektroskopie mit induktiv gekoppeltem Plasma-(ICP-OES)-Verfahren weniger als 0,01 mg/kg als Ni beträgt;

- eine Paraphenylendiaminkonzentration, welche gemäß Lösungsmittelextraktion und/oder Gaschromatographie-Massenspektrometrie (GC-MS) und/oder Flüssigkeitschromatographie mit Ultraviolett-(LC-UV)-Bestimmung weniger als 0,5 mg/kg beträgt;

- eine Organozinnverbindungen-Konzentration, welche gemäß CEN ISO/TS 16179:2012 weniger als 0,02 mg/kg als Ni beträgt;

- eine Azofarbstoffe-Konzentration, welche gemäß UNI EN 14362 weniger als 5 mg/kg beträgt.

8. Verfahren oder Schuhwerk nach einem der vorhergehenden Ansprüche, wobei die vordefinierte Liste mit allergenen Materialien und entsprechenden Maximalkonzentrationen der Oberschale (1), insbesondere der Oberschale (1), der Fütterung (4) und der Innensohle (2), des Schuhwerks (10) ferner umfasst:

- aromatische Amine, wobei die aromatischen Amine gemäß EN 14362-1:2017 oder EN 14362-2:2003/AC:2005 oder EN 14362-3:2017 höchstens in einer Konzentration von weniger als 5 mg/kg vorhanden sind, wobei die aromatischen Amine eines oder mehrere umfassen aus:

- Benzidin, wobei das Benzidin höchstens mit einer Konzentration von weniger als 5 mg/kg vorhanden ist;
- o-Anisidin, wobei das o-Anisidin höchstens mit einer Konzentration von weniger als 5 mg/kg vorhanden ist;
- o-Aminoazotoluol, wobei das o-Aminoazotoluol höchstens mit einer Konzentration von weniger als 5 mg/kg vorhanden ist;
- o-Toluidin, wobei das o-Toluidin höchstens mit einer Konzentration von weniger als 5 mg/kg vorhanden ist;
- p-Kresidin, wobei das p-Kresidin höchstens mit einer Konzentration von weniger als 5 mg/kg vorhanden ist;
- p-Chloranilin, wobei das Chloranilin höchstens mit einer Konzentration von weniger als 5 mg/kg vorhanden ist;
- 2-Amino-4-Nitrotoluol, wobei das 2-Amino-4-Nitrotoluol höchstens mit einer Konzentration von weniger als 5 mg/kg vorhanden ist;
- 2-Naphthylamin, wobei das 2-Naphthylamin höchstens mit einer Konzentration von weniger als 5 mg/kg vorhanden ist;
- 2,4-Toluylendiamin, wobei das 2,4-Toluylendiamin höchstens mit einer Konzentration von weniger als 5 mg/kg vorhanden ist;
- 2,4-Xylidin, wobei das 2,4-Xylidin höchstens mit einer Konzentration von weniger als 5 mg/kg vorhanden ist;
- 2,6-Xylidin, wobei das 2,6-Xylidin höchstens mit einer Konzentration von weniger als 5 mg/kg vorhanden ist;
- 3,3'-Dimethylbenzidin, wobei das 3,3'-Dimethylbenzidin höchstens mit einer Konzentration von weniger als 5 mg/kg vorhanden ist;
- 3,3'-Dichlorbenzidin, wobei das 3,3'-Dichlorbenzidin höchstens mit einer Konzentration von weniger als 5 mg/kg vorhanden ist;
- 3,3'-Dimethoxybenzidin, wobei das 3,3'-Dimethoxybenzidin höchstens mit einer Konzentration von weniger als 5 mg/kg vorhanden ist;
- 3,3'-Dimthyl-4,4'-Diaminodiphenylmethan, wobei das 3'-Dimthyl-4,4'-Diaminodiphenylmethan höchstens mit einer Konzentration von weniger als 5 mg/kg vorhanden ist;
- 4-Aminodiphenyl, wobei das 4-Aminodiphenyl höchstens mit einer Konzentration von weniger als 5 mg/kg vorhanden ist;
- 4-Chlor-O-Toluin, wobei das 4-Chlor-O-Toluin höchstens mit einer Konzentration von weniger als 5 mg/kg vorhanden ist;
- 4,4'-Diaminodiphenylmethan, wobei das 4,4'-Diaminodiphenylmethan höchstens mit einer Konzentration von weniger als 5 mg/kg vorhanden ist;
- 4,4'-Methylen-Bis-(2-Chloranilin), wobei das 4,4'-Methylen-Bis-(2-Chloranilin) höchstens mit einer Konzentration von weniger als 5 mg/kg vorhanden ist;
- 4,4'-Thiodianilin, wobei das Thiodianilin höchstens mit einer Konzentration von weniger als 5 mg/kg vorhanden ist;
- 4,4'-Oxydianilin, wobei das 4,4'-Oxydianilin höchstens mit einer Konzentration von weniger als 5 mg/kg vorhanden ist;
- 2,4,5-Trimethylanilin, wobei das 2,4,5-Trimethylanilin höchstens mit einer Konzentration von weniger als 5 mg/kg vorhanden ist;
- 4-Aminoazobenzol, wobei das 4-Aminoazobenzol höchstens mit einer Konzentration von weniger als 5 mg/kg vorhanden ist;

- Schwermetall(e), wobei das/die Schwermetall(e) gemäß DIN 54233-3:2010 und/oder EN1122:2001 und/oder Säureaufschluss und/oder Emissionsspektroskopie mit induktiv gekoppeltem Plasma-(ICP-OES)-Verfahren höchstens in einer Konzentration, insbesondere lösbarer Schwermetallkonzentration, von weniger als 0,01 mg/kg vorhanden ist/sind, optional wobei die Schwermetalle wenigstens eines umfassen aus einer:

    - Arsen-(As)-Konzentration von weniger als 0,01 mg/kg;
    - Kadmium-(Cd)-Konzentration von weniger als 0,01 mg/kg;
    - Chrom-Konzentration von weniger als 0,01 mg/kg;
    - Kobalt-(Co)-Konzentration von weniger als 0,01 mg/kg;
    - Blei-(Pb)-Konzentration von weniger als 0,01 mg/kg;
    - Quecksilber-(Hg)-Konzentration von weniger als 0,01 mg/kg;
    - Nickel-(Ni)-Konzentration von weniger als 0,01 mg/kg;

optional wobei das Schuhwerk (10) ferner einen Kragen (6) und/oder eine Verstärkung (5) umfasst, welcher/welche die aromatischen Amine gemäß EN 14362-1:2017 oder EN 14362-2:2003/AC:2005 oder EN 14362-3:2017 in einer Konzentration von weniger als 5 mg/kg, und/oder das/die Schwermetall(e) gemäß DIN 54233-3:2010 und/oder EN 1122:2001 und/oder Säureaufschluss und/oder Emissionsspektroskopie mit induktiv gekoppeltem Plasma-(ICP-OES)-Verfahren in einer Konzentration von weniger als 0,1 mg/kg umfasst/umfassen.

9. Verfahren oder Schuhwerk nach einem der vorhergehenden Ansprüche, wobei wenigstens eine aus der Oberschale (1), der Fütterung (4) und der Innensohle (2) des Schuhwerks (10) einen pH-Wert aufweist, welcher gemäß EN ISO 3071:2020 zwischen 5 und 7,5 umfasst ist, insbesondere einen pH-Wert, welcher zwischen 5,5 und 7 umfasst ist.

10. Verfahren oder Schuhwerk nach einem der vorhergehenden Ansprüche, wobei die Liste mit allergenen Materialien und entsprechenden Konzentrationen und/oder die zweite verschiedene Liste mit allergenen Materialien umfasst:

- einen Mercapto-Mix, wobei der Mercapto-Mix gemäß Lösungsmittelextraktion und Bestimmung nach HPLC-UV höchstens mit einer Konzentration von weniger als 0,1 mg/kg vorhanden ist;
- einen Carba-Mix, wobei der Carba-Mix gemäß ... höchstens mit einer Konzentration von weniger als 0,1 mg/kg vorhanden ist;
- einen Thiuram-Mix, wobei der Thiuram-Mix höchstens mit einer Konzentration von weniger als 0,1 mg/kg vorhanden ist;
- Mercaptobenzothiazol, wobei das Mercaptobenzothiazol höchstens mit einer Konzentration von weniger als 0,1 mg/kg vorhanden ist;
- gemischte Dialkil-Thioharnstoffe, wobei die gemischten Dialkil-Thioharnstoffe höchstens mit einer Konzentration von weniger als 0,1 mg/kg vorhanden sind;
- einen Schwarzer-Gummi-Mix, wobei der Schwarzer-Gummi-Mix höchstens mit einer Konzentration von weniger als 0,1 mg/kg vorhanden ist;
- einen Paraben-Mix, wobei der Paraben-Mix höchstens mit einer Konzentration von weniger als 0,1 mg/kg vorhanden ist.

11. Verfahren oder Schuhwerk nach einem der vorhergehenden Ansprüche, wobei das Schuhwerk (10) ferner eines oder mehrere umfasst aus:

- einer Verstärkung (5) gemäß der vordefinierten Liste mit allergenen Materialien und entsprechenden Maximalkonzentrationen;
- einem Kragen (6) gemäß der vordefinierten Liste mit allergenen Materialien und entsprechenden Maximalkonzentrationen.

12. Verfahren oder Schuhwerk nach einem der vorhergehenden Ansprüche, wobei die durchlässige Struktur gemäß EN 13515:2001/ISO 17 699:2003 eine Wasserdampfabsorption aufweist, welche gleich wie oder größer als 1 mg/cm$^2$ ist, insbesondere größer als 1,5 mg/cm$^2$ ist.

13. Verfahren oder Schuhwerk nach einem der vorhergehenden Ansprüche, wobei die Oberschale aufweist:

- eine Bruchfestigkeit, welche gemäß UNI EN ISO 13934-1:2013 größer als oder gleich wie 500 N/50 mm ist, insbesondere größer als 1000 N/50 mm ist; und/oder
- eine Bruchdehnung bei Versagen, welche gemäß UNI EN ISO 13934-1:2013 größer als oder gleich wie 15% ist, insbesondere größer als 40% ist, im Besonderen größer als 55% ist.

14. Verfahren oder Schuhwerk nach einem der vorhergehenden Ansprüche, wobei die Hautverträglichkeit, welche durch den Wachstumshemmungsprozentsatz definiert ist, anhand der folgenden Formel berechnet wird:

$$\%WH = 100 - 100 \cdot \frac{(OD_{570nm} sample) - (OD_{570nm} blank)}{(OD_{570nm} control) - (OD_{570nm} blank)}$$

wobei:

OD570nm = bei 570 nm Wellenlänge quantifizierte optische Dichte;
OD570nm Sample = durchschnittliche Extinktion einer Probenverdünnung aus 3 parallelen Quellen;
OD570nm Blank = durchschnittliche Extinktion einer Blank-Verdünnung aus 3 parallelen Quellen, wobei die Blank-Verdünnung ohne Zellen durchgeführt wird;
OD570nm Control = Durchschnittliche Extinktion der Lösungsmittelkontrolle aus 3 parallelen Quellen.

15. Verfahren oder Schuhwerk nach einem der vorhergehenden Ansprüche, wobei die Oberschale (1), insbesondere die Oberschale (1) und die Fütterung (4), basiert/basieren auf und insbesondere hergestellt ist/sind aus: Nylon oder Polyester oder bio-natürlichen Stoffen, beispielsweise organische Baumwolle oder Hanf, oder einer Kombination davon, und keine Materialien tierischen Ursprungs umfasst/umfassen.

**Revendications**

1. Méthode de prévention d'une dermatite allergique de contact (DAC) au niveau du pied d'un utilisateur par le port d'une chaussure (10) hypoallergénique, ladite chaussure comprenant au moins une tige (1), une semelle intérieure (2) et une semelle extérieure (3),

   - au moins la tige (1) et la semelle intérieure (2) présentent une structure hypoallergénique selon une liste prédéfinie de matériaux allergéniques et correspondant à des concentrations maximales, ladite liste comprenant :

      ◦ du sel de chrome, le sel de chrome étant présent au plus sous une concentration inférieure à 0,01 mg/kg selon la norme DIN 54233-3:2010 ;
      ◦ du fumarate de diméthyle, le fumarate de diméthyle étant présent au plus sous une concentration inférieure à 0,01 mg/kg selon la norme EN ISO 16186:2012 ;
      ◦ du para butylphénol formaldéhyde tertiaire, le para butylphénol formaldéhyde tertiaire étant présent au plus sous une concentration inférieure à 5 mg/kg selon la norme EN ISO 14184-1:2011 ;
      ◦ du formaldéhyde, le formaldéhyde étant présent au plus sous une concentration inférieure à 5 mg/kg selon la norme EN ISO 14184-1:2011 ;
      ◦ du chrome, soluble total, le chrome étant présent au plus sous une concentration inférieure à 0,01 mg/kg selon la norme DIN 54233-3:2010 ;

   - ladite chaussure (10), en particulier au moins la tige (1), définissant une structure perméable ayant :

      ◦ une perméabilité à la vapeur d'eau supérieure ou égale à 2,0 mg/(cm$^2$·h), en particulier supérieure à 1,3 mg/(cm$^2$·h), selon la norme EN 13515:2001/ISO 17 699:2003 ; et/ou
      ◦ un coefficient de vapeur d'eau supérieur ou égal à 20 mg/cm$^2$, en particulier supérieur à 50 mg/cm$^2$, selon la norme EN 13515:2001/ISO 17 699:2003 ;

   - et au moins la semelle intérieure (2) ayant, éventuellement au moins la tige (1) et la semelle intérieure (2) ayant, une dermocompatibilité définie par un pourcentage d'inhibition de croissance inférieur ou égal à 30 %, en particulier inférieur à 25 %, selon la norme DIN EN ISO 10993, DIN EN ISO 10993-5.

2. Chaussure (10) hypoallergénique comprenant au moins une tige (1), une semelle intérieure (2) et une semelle extérieure (3),

- au moins la tige (1) et la semelle intérieure (2) ayant une structure hypoallergénique selon une liste prédéfinie de matériaux allergéniques et des concentrations maximales correspondantes, ladite liste comprenant :

◦ du sel de chrome, le sel de chrome étant présent au plus sous une concentration inférieure à 0,01 mg/kg selon la norme DIN 54233-3:2010 ;
◦ du fumarate de diméthyle, le fumarate de diméthyle étant présent au plus sous une concentration inférieure à 0,01 mg/kg selon la norme EN ISO 16186:2012 ;
◦ du para butylphénol formaldéhyde tertiaire, le para butylphénol formaldéhyde tertiaire étant présent au plus sous une concentration inférieure à 5 mg/kg selon la norme EN ISO 14184-1:2011 ;
◦ du formaldéhyde, le formaldéhyde étant présent au plus sous une concentration inférieure à 5 mg/kg selon la norme EN ISO 14184-1:2011 ;
◦ du chrome, soluble total, le chrome étant présent au plus sous une concentration inférieure à 0,01 mg/kg selon la norme DIN 54233-3:2010 ;

- ladite chaussure (10), en particulier au moins la tige (1), définissant une structure perméable ayant :

◦ une perméabilité à la vapeur d'eau supérieure ou égale à 2,0 mg/(cm$^2$·h), en particulier supérieure à 1,3 mg/(cm$^2$·h), selon la norme EN 13515:2001/ISO 17 699:2003 ; et/ou
◦ un coefficient de vapeur d'eau supérieur ou égal à 20 mg/cm$^2$, en particulier supérieur à 50 mg/cm$^2$, selon la norme EN 13515:2001/ISO 17 699:2003 ;

- et au moins la semelle intérieure (2) ayant, éventuellement au moins la tige (1) et la semelle intérieure (2) ayant, une dermocompatibilité définie par un pourcentage d'inhibition de croissance inférieur ou égal à 30 %, en particulier inférieur à 25 %, selon la norme DIN EN ISO 10993, DIN EN ISO 10993-5.

3. Méthode ou chaussure selon l'une quelconque des revendications précédentes, la chaussure (10) incluant en outre une doublure (4) comprenant la structure hypoallergénique selon ladite liste de concentrations maximales prédéfinies de matériaux allergéniques, ladite doublure (4) contactant le pied de l'utilisateur lorsque la chaussure (10) hypoallergénique se trouve en cours d'utilisation,
la doublure (4) ayant une dermocompatibilité définie par un pourcentage d'inhibition de croissance inférieur ou égal à 30 %, en particulier inférieur à 25 %, selon la norme DIN EN ISO 10993, DIN EN ISO 10993-5.

4. Méthode ou chaussure selon l'une quelconque des revendications précédentes, ladite liste prédéfinie de matériaux allergéniques et les concentrations maximales correspondantes incluant en outre :

◦ du dichromate de potassium, le dichromate de potassium étant présent au plus sous une concentration inférieure à 0,01 mg/kg sous la forme de Cr VI selon des procédés de digestion par un acide et de spectroscopie d'émission optique avec plasma à couplage inductif (ICP-OES) ;
◦ du chlorure de cobalt, le chlorure de cobalt étant présent au plus sous une concentration inférieure à 0,01 mg/kg sous la forme de Co selon des procédés de digestion par un acide et de spectroscopie d'émission optique avec plasma à couplage inductif (ICP-OES) ;
◦ du sulfate de nickel, le sulfate de nickel étant présent au plus sous une concentration inférieure à 0,01 mg/kg sous la forme de Ni selon des procédés de digestion par un acide et de spectroscopie d'émission optique avec plasma à couplage inductif (ICP-OES).

5. Méthode ou chaussure selon l'une quelconque des revendications précédentes, ladite liste de matériaux allergéniques de la tige (1) et de la semelle intérieure (2), éventuellement de la doublure (4), de la semelle intérieure (2) et préférablement également de la tige (1), incluant en outre :

◦ de la paraphénylènediamine, la paraphénylènediamine étant présente au plus sous une concentration inférieure à 0,5 mg/kg par extraction au solvant et/ou détermination par chromatographie en phase gazeuse-spectrométrie de masse (GC-MS) et/ou par chromatographie en phase liquide couplée à un détecteur par ultraviolets (LC-UV) ;
◦ un composé d'organoétain, le composé d'organoétain étant présent au plus sous une concentration inférieure à 0,02 mg/kg sous la forme de Ni selon la norme CEN ISO/TS 16179:2012 ;
◦ des colorants azoïques, les colorants azoïques étant présents au plus sous une concentration inférieure à 5 mg/kg selon la norme UNI EN 14362.

**6.** Méthode ou chaussure selon l'une quelconque des revendications précédentes, la semelle extérieure (3) ayant une dermocompatibilité supérieure à 30 % et:

- une structure comprenant la même liste prédéfinie de matériaux allergéniques et de concentrations maximales correspondantes de la tige (1) et de la semelle intérieure (2), en particulier de la tige (1), de la semelle intérieure (2) et de la doublure (4), en particulier ladite liste de matériaux allergéniques comprenant toutes ou au moins une substance parmi:

◦ des sels de chrome, les sels de chrome étant présents au plus sous une concentration inférieure à 0,01 mg/kg selon la norme DIN 54233-3:2010;
◦ du fumarate de diméthyle, ledit fumarate de diméthyle étant présent au plus sous une concentration inférieure à 0,01 mg/kg selon la norme EN ISO 16186:2012;
◦ du para butylphénol formaldéhyde tertiaire, ledit para butylphénol formaldéhyde tertiaire étant présent au plus sous une concentration inférieure à 5 mg/kg selon la norme EN ISO 14184-1:2011 ;
◦ du formaldéhyde, ledit formaldéhyde étant présent au plus sous une concentration inférieure à 5 mg/kg selon la norme EN ISO 14184-1:2011;
◦ une concentration soluble totale en chrome inférieure à 0,01 mg/kg selon la norme DIN 54233-3:2010;
◦ du dichromate de potassium, ledit dichromate de potassium étant présent au plus sous une concentration inférieure à 0,01 mg/kg sous la forme de Cr VI selon des procédés de digestion par un acide et de spectroscopie d'émission optique avec plasma à couplage inductif (ICP-OES);
◦ du chlorure de cobalt, ledit chlorure de cobalt étant présent au plus sous une concentration inférieure à 0,01 mg/kg sous la forme de Co selon des procédés de digestion par un acide et de spectroscopie d'émission optique avec plasma à couplage inductif (ICP-OES);
◦ du sulfate de nickel, ledit sulfate de nickel étant présent au plus sous une concentration inférieure à 0,01 mg/kg sous la forme de Ni selon des procédés de digestion par un acide et de spectroscopie d'émission optique avec plasma à couplage inductif (ICP-OES),
◦ de la paraphénylènediamine, ladite paraphénylènediamine étant présente au plus sous une concentration inférieure à 0,5 mg/kg par extraction au solvant et/ou détermination par chromatographie en phase gazeuse-spectrométrie de masse (GC-MS) et/ou chromatographie en phase liquide couplée à un détecteur par ultraviolets (LC-UV);
◦ des composés d'organoétain, lesdits composés d'organoétain étant présents au plus sous une concentration inférieure à 0,02 mg/kg sous la forme de Ni selon la norme CEN ISO/TS 16179:2012;
◦ des colorants azoïques, lesdits colorants azoïques étant présents au plus sous une concentration inférieure à 5 mg/kg selon la norme UNI EN 14362;

ou
- une seconde liste distincte de matériaux allergéniques incluant toutes ou au moins une substance parmi:

◦ des sels de chrome, lesdits sels de chrome étant présents au plus sous une concentration inférieure à 0,01 mg/kg selon la norme DIN 54233-3:2010;
◦ du fumarate de diméthyle, ledit fumarate de diméthyle étant présent au plus sous une concentration inférieure à 0,01 mg/kg selon la norme EN ISO 16186:2012;
◦ du para butylphénol formaldéhyde tertiaire, ledit para butylphénol formaldéhyde tertiaire étant présent au plus sous une concentration inférieure à 5 mg/kg selon la norme EN ISO 14184-1:2011;
◦ du formaldéhyde, ledit formaldéhyde étant présent au plus sous une concentration inférieure à 5 mg/kg selon la norme EN ISO 14184-1:2011;
◦ du chrome, soluble total, ledit chrome soluble total étant présent au plus sous une concentration inférieure à 0,01 mg/kg selon la norme DIN 54233-3:2010.

**7.** Méthode ou chaussure selon la revendication précédente 6, ladite seconde liste distincte de matériaux allergéniques incluant en outre :

- une concentration de dichromate de potassium inférieure à 0,01 mg/kg sous la forme de Cr VI selon des procédés de digestion par un acide et de spectroscopie d'émission optique avec plasma à couplage inductif (ICP-OES) ;
- une concentration de chlorure de cobalt inférieure à 0,01 mg/kg sous la forme de Co selon des procédés de digestion par un acide et de spectroscopie d'émission optique avec plasma à couplage inductif (ICP-OES) ;
- une concentration de sulfate de nickel inférieure à 0,01 mg/kg sous la forme de Ni selon des procédés de

EP 4 035 552 B1

digestion par un acide et de spectroscopie d'émission optique avec plasma à couplage inductif (ICP-OES) ;
- une concentration de paraphénylènediamine inférieure à 0,5 mg/kg par extraction au solvant et/ou détermination par chromatographie en phase gazeuse-spectrométrie de masse (GC-MS) et/ou chromatographie en phase liquide couplée à un détecteur par ultraviolets (LC-UV) ;
- une concentration des composés d'organoétain inférieure à 0,02 mg/kg sous la forme de Ni selon la norme CEN ISO/TS 16179:2012 ;
- une concentration des colorants azoïques inférieure à 5 mg/kg selon la norme UNI EN 14362.

8. Méthode ou chaussure selon l'une quelconque des revendications précédentes, la liste prédéfinie de matériaux allergéniques et les concentrations maximales correspondantes de la tige (1), en particulier de la tige (1), de la doublure (4) et de la semelle intérieure (2), de la chaussure (10) incluant en outre :

- des amines aromatiques, lesdites amines aromatiques étant présentes au plus sous une concentration inférieure à 5 mg/kg selon la norme EN 14362-1:2017 ou EN 14362-2:2003/AC:2005 ou EN 14362-3:2017, les amines aromatiques comprenant l'un·e ou plusieurs parmi :

  ◦ la benzidine, ladite benzidine étant présente au plus sous une concentration inférieure à 5 mg/kg;
  ◦ la o-anisidine, ladite o-anisidine étant présente au plus sous une concentration inférieure à 5 mg/kg;
  ◦ le o-aminoazotoluène, ledit o-aminoazotoluène étant présent au plus sous une concentration inférieure à 5 mg/kg;
  ◦ la o-toluidine, ladite o-toluidine étant présente au plus sous une concentration inférieure à 5 mg/kg;
  ◦ la p-crésidine, ladite p-crésidine étant présente au plus sous une concentration inférieure à 5 mg/kg;
  ◦ la p-chloroaniline, ladite chloroaniline étant présente au plus sous une concentration inférieure à 5 mg/kg;
  ◦ le 2-amino-4-nitrotoluène, ledit 2-amino-4-nitrotoluène étant présent au plus sous une concentration inférieure à 5 mg/kg;
  ◦ la 2-naphtylamine, ladite 2-naphtylamine étant présente au plus sous une concentration inférieure à 5 mg/kg;
  ◦ la 2,4-toluylènediamine, ladite 2,4-toluylènediamine étant présente au plus sous une concentration inférieure à 5 mg/kg;
  ◦ la 2,4-xylidine, ladite 2,4-xylidine étant présente au plus sous une concentration inférieure à 5 mg/kg;
  ◦ la 2,6-xylidine, ladite 2,6-xylidine étant présente au plus sous une concentration inférieure à 5 mg/kg;
  ◦ la 3,3'-diméthylbenzidine, ladite 3,3'-diméthylbenzidine étant présente au plus sous une concentration inférieure à 5 mg/kg;
  ◦ la 3,3'-dichlorobenzidine, ladite 3,3'-dichlorobenzidine étant présente au plus sous une concentration inférieure à 5 mg/kg;
  ◦ la 3,3'-diméthoxybenzidine, ladite 3,3'-diméthoxybenzidine étant présente au plus sous une concentration inférieure à 5 mg/kg;
  ◦ le 3,3'-diméthyl-4,4'-diaminodiphénylméthane, ledit 3'-diméthyl-4,4'-diaminodiphénylméthane étant présent au plus sous une concentration inférieure à 5 mg/kg;
  ◦ le 4-aminodiphényle, ledit 4-aminodiphényle étant présent au plus sous une concentration inférieure à 5 mg/kg ;
  ◦ la 4-chloro-O-toluine, ladite 4-chloro-O-toluine étant présente au plus sous une concentration inférieure à 5 mg/kg;
  ◦ le 4,4'-diaminodiphénylméthane, ledit 4,4'-diaminodiphénylméthane étant présent au plus sous une concentration inférieure à 5 mg/kg;
  ◦ la 4,4'-methylène-bis-(2-chloroaniline), ladite 4,4'-methylène-bis-(2-chloroaniline) étant présente au plus sous une concentration inférieure à 5 mg/kg;
  ◦ la 4,4'-thiodianiline, ladite thiodianiline étant présente au plus sous une concentration inférieure à 5 mg/kg ;
  ◦ la 4,4'-oxydianiline, ladite 4,4'-oxydianiline étant présente au plus sous une concentration inférieure à 5 mg/kg ;
  ◦ la 2,4,5-triméthylaniline, ladite 2,4,5-triméthylaniline étant présente au plus sous une concentration inférieure à 5 mg/kg ;
  ◦ le 4-aminoazobenzène, ledit 4-aminoazobenzène étant présent au plus sous une concentration inférieure à 5 mg/kg ;

- un(des) métal(ux) lourd(s), ledit(lesdits) métal(ux) lourd(s) étant présent(s) au plus sous une concentration, en particulier une concentration en métaux lourds solubles, inférieure à 0,1 mg/kg selon la norme DIN 54233-3:2010 et/ou EN1122:2001 et/ou procédé de digestion par un acide et/ou de spectroscopie d'émission optique avec plasma à couplage inductif (ICP-OES), éventuellement lesdits métaux lourds comprenant au moins l'une entre :

- ◦ une concentration en arsenic (As) inférieure à 0,01 mg/kg ;
- ◦ une concentration en cadmium (Cd) inférieure à 0,01 mg/kg ;
- ◦ une concentration en chrome inférieure à 0,01 mg/kg ;
- ◦ une concentration en cobalt (Co) inférieure à 0,01 mg/kg ;
- ◦ une concentration en plomb (Pb) inférieure à 0,01 mg/kg ;
- ◦ une concentration en mercure (Hg) inférieure à 0,01 mg/kg ;
- ◦ une concentration en nickel (Ni) inférieure à 0,01 mg/kg ;

éventuellement la chaussure (10) incluant en outre un bracelet (6) et/ou un renforcement (5) comprenant lesdites amines aromatiques sous une concentration inférieure à 5 mg/kg selon la norme EN 14362-1:2017 ou EN 14362-2:2003/AC:2005 ou EN 14362-3:2017, et/ou ledit(lesdits) métal(ux) lourd(s) sous une concentration inférieure à 0,1 mg/kg selon la norme DIN 54233-3:2010 et/ou EN1 122:2001 et/ou un procédé de digestion par un acide et/ou de spectroscopie d'émission optique avec plasma à couplage inductif (ICP-OES).

9. Méthode ou chaussure selon l'une quelconque des revendications précédentes, au moins l'une entre la tige (1), la doublure (4) et la semelle intérieure (2) de la chaussure (10) ayant une valeur de pH comprise entre 5 et 7,5 selon la norme EN ISO 3071:2020, en particulier une valeur de pH comprise entre 5,5 et 7.

10. Méthode ou chaussure selon l'une quelconque des revendications précédentes, la liste des matériaux allergéniques et des concentrations correspondantes et/ou la seconde liste distincte des matériaux allergéniques comprenant :

- un mélange mercapto, ledit mélange mercapto étant présent au plus sous une concentration inférieure à 0,1 mg/kg par extraction au solvant et détermination par *CLHP-UV* ;
- un mélange carba, ledit mélange carba étant présent au plus sous une concentration inférieure à 0,1 mg/kg en fonction de ... ;
- un mélange thiuram, ledit mélange thiuram étant présent au plus sous une concentration inférieure à 0,1 mg/kg ;
- du mercaptobenziotaziolo, ledit mercaptobenziotaziolo étant présent au plus sous une concentration inférieure à 0,1 mg/kg ;
- des dialkyl thiourées mixtes, lesdites dialkyl thiourées mixtes étant présentes au plus sous une concentration inférieure à 0,1 mg/kg ;
- un mélange de caoutchouc noir, ledit mélange de caoutchouc noir étant présent au plus sous une concentration inférieure à 0,1 mg/kg ;
- un mélange de parabènes, ledit mélange de parabeni étant présent au plus sous une concentration inférieure à 0,1 mg/kg.

11. Méthode ou chaussure selon l'une quelconque des revendications précédentes, la chaussure (10) comprenant en outre l'un ou plusieurs parmi:

- un renforcement (5) selon ladite liste prédéfinie de matériaux allergéniques et les concentrations maximales correspondantes;
- un bracelet (6) selon ladite liste prédéfinie de matériaux allergéniques et les concentrations maximales correspondantes.

12. Méthode ou chaussure selon l'une quelconque des revendications précédentes, la structure perméable ayant une absorption de vapeur d'eau supérieure ou égale à 1 mg/cm$^2$, en particulier supérieure à 1,5 mg/cm$^2$, selon la norme EN 13515:2001/ISO 17 699:2003.

13. Méthode ou chaussure selon l'une quelconque des revendications précédentes, la tige ayant :

- une résistance ultime supérieure ou égale à 500 N/50 mm selon la norme UNI EN ISO13934-1:2013, en particulier supérieure à 1 000 N/50 mm ; et/ou
- un allongement ultime à la rupture supérieur ou égal à 15 % selon la norme UNI EN ISO 13934-1:2013, en particulier supérieur à 40 %, plus particulièrement supérieur à 55 %.

14. Méthode ou chaussure selon l'une quelconque des revendications précédentes, la dermocompatibilité définie par un pourcentage d'inhibition de croissance étant calculée par la formule suivante :

$$\%WH = 100 - 100 \cdot \frac{(OD_{570nm}sample) - (OD_{570nm}blank)}{(OD_{570nm}control) - (OD_{570nm}blank)}$$

d.o.$_{570nm}$ = densité optique quantifiée à des longueurs d'ondes de 570 nm ;
d.o.$_{570nm}$ échantillon = extinction moyenne d'un échantillon de dilution de 3 puits parallèles ;
d.o.$_{570nm}$ à blanc = extinction moyenne d'une dilution à blanc de 3 puits parallèles, la dilution à blanc étant exempte de cellules ;
d.o.$_{570nm}$ témoin = extinction moyenne du témoin solvant à partir de 3 puits parallèles.

**15.** Méthode ou chaussure selon l'une quelconque des revendications précédentes, la tige (1), en particulier la tige (1) et la doublure (4), étant à base, et particulièrement constituées, de nylon ou de polyester ou de textiles naturels d'origine biologique, tels que du coton et du chanvre biologique, ou une combinaison de ceux-ci et comprend(comprennent)/ne comprend(comprennent) aucuns matériaux d'origine animale.

FIG.1

FIG.2

## REFERENCES CITED IN THE DESCRIPTION

**Patent documents cited in the description**

- US 2011162239 A1